# FASCICULE DE BREVET EUROPEEN

(11) **EP 3 636 229 B1**
(45) Date de publication et mention de la délivrance du brevet: **07.07.2021**
(21) Numéro de dépôt: 19202777.9
(22) Date de dépôt: 11.10.2019
(51) Int. Cl.: A61F 7/00

(54) **MACHINE ET SYSTEME POUR UN TRAITEMENT PAR CRYOTHERAPIE D'UNE ZONE D'UN CORPS HUMAIN OU ANIMAL**
MASCHINE UND SYSTEM ZUR KRYOTHERAPIE BEHANDLUNG EINER ZONE EINES MENSCHLICHEN ODER TIERISCHEN KÖRPERS
MACHINE AND SYSTEM FOR CRYOTHERAPY TREATMENT OF A ZONE OF A HUMAN OR ANIMAL BODY

(30) Priorité: 12.10.2018 FR 1859471
(43) Date de publication de la demande: 15.04.2020
(73) Titulaire: Carabalona, Cédric, 13840 Rognes (FR)
(72) Inventeur: Carabalona, Cédric, 13840 Rognes (FR)
(74) Mandataire: Roman, Alexis

(56) Documents cités:
- EP-A1- 3 021 802
- EP-A2- 2 863 850
- US-A1- 2018 193 186
- US-B1- 6 551 347

## Description

### Domaine technique de l'invention.

L'invention a pour objet une machine et un système pour le traitement par cryothérapie d'une zone d'un corps humain ou animal. Elle concerne également des procédés de fonctionnement d'une telle machine.

Elle concerne le domaine technique des machines adaptées pour un traitement par cryothérapie, consistant à appliquer du froid sur une zone du corps humain ou animal.

### État de la technique.

La cryothérapie est une technique de traitement bien connue notamment utilisée pour diminuer un œdème ou un gonflement, réduire la douleur, soulager une inflammation, permettre une récupération musculaire, etc.

Généralement, un liquide froid est mis en circulation dans une enveloppe adaptée pour être installée sur la zone du corps à traiter. Une machine de cryothérapie permet de produire le liquide froid et le distribuer à l'enveloppe.

Ces machines sont biens connues de l'art antérieur et par exemple décrites dans les documents brevets EP2490633 (COOLSYSTEMS) ou EP3308753 (COOLSYSTEMS).

On connait plus particulièrement par les documents brevets US2010/0145421 (TOMLINSON) et US2010/0139294 (LOWE) une machine de cryothérapie comportant :
- un réservoir de liquide présentant une entrée et une sortie,
- une pompe présentant une entrée d'aspiration du liquide et une sortie de refoulement du liquide, laquelle pompe est située en aval du réservoir,
- une unité de réfrigération du liquide, laquelle unité comporte une entrée et une sortie,
- un circuit hydraulique présentant des lignes de connexion pour connecter : le réservoir, la pompe et l'unité de réfrigération, lequel circuit comporte :
   ∘ une première ligne reliant la sortie de refoulement de la pompe à l'entrée de l'unité de réfrigération via une soupape de dérivation,
   ∘ une deuxième ligne reliant la sortie de l'unité de réfrigération à l'entrée du réservoir,
   ∘ une troisième ligne reliant la sortie du réservoir à l'entrée d'aspiration de la pompe.
- un dispositif de connexion adapté pour mettre en communication hydraulique le circuit avec l'enveloppe.

Le document brevet EP3021802 (PHYSIOLAB) décrit également une machine de cryothérapie conforme au préambule de la revendication 1.

Ce type de machines connues de l'art antérieur ne permet pas un contrôle optimal de la température du liquide circulant dans l'enveloppe. Cette température est pourtant un facteur essentiel pour l'efficacité du traitement.

Dans les machines précitées, un contrôle de la température est réalisé dans le réservoir de liquide. Toutefois, entre la sortie du réservoir et le dispositif de connexion, un certain nombre d'éléments, dont la pompe, sont susceptibles de réchauffer le liquide. Il en résulte que la température du liquide circulant dans l'enveloppe est généralement supérieure à la température mesurée dans le réservoir, de sorte que l'efficacité du traitement n'est pas optimale.

De plus, au début d'une séance, la température du liquide contenu dans le réservoir est généralement supérieure à une température de traitement. Par exemple, la température du liquide dans le réservoir peut être de 20°C alors que la température cible de traitement est de 10°C. Compte tenu de l'inertie thermique du liquide, l'unité de réfrigération met un certain temps (par exemple 10 minutes) à refroidir le liquide pour qu'il atteigne la température cible de traitement. Le praticien fait généralement circuler le liquide dans l'enveloppe pendant cet intervalle de temps. Une séance de cryothérapie durant généralement entre 15 minutes et 20 minutes, les effets bénéfiques de cette séance sont amputés de toute cette période de refroidissement.

En outre, pendant une séance de cryothérapie, seule une fraction du volume liquide refoulé par la pompe circule directement vers l'unité de réfrigération. L'autre partie circule dans l'enveloppe avant de retourner dans l'unité de réfrigération. Il en résulte que le refroidissement du liquide pendant la séance est relativement long. En pratique, l'unité de réfrigération est activée en permanence durant la séance, ce qui peut être relativement coûteux.

L'invention vise à remédier à cet état des choses. En particulier, un objectif de l'invention est de proposer une machine de cryothérapie dont la conception permet un traitement optimale de la zone du corps.

Un autre objectif de l'invention est de proposer une machine de cryothérapie permettant un meilleur contrôle de la température du liquide circulant dans l'enveloppe.

Encore un autre objectif de l'invention est de proposer un procédé de fonctionnement d'une machine de cryothérapie permettant d'optimiser la durée d'efficacité de la séance.

Un objectif supplémentaire de l'invention est de proposer un procédé de fonctionnement d'une machine de cryothérapie permettant de refroidir très rapidement et à moindre coûts le liquide.

### Divulgation de l'invention.

La solution proposée par l'invention est une machine conforme à la revendication 1.

La sortie du réservoir est maintenant connectée directement à la sortie du circuit hydraulique, la pompe étant située en amont dudit réservoir. La pompe n'est donc plus susceptible de réchauffer de manière intempestive le liquide qui va circuler dans l'enveloppe, de sorte que la température dudit liquide est mieux contrôlée. En outre, on peut faire circuler le liquide en circuit fermé, à l'intérieur de la machine, pour l'amener à la bonne température avant de débuter une séance. Ainsi, dès que la séance commence, le liquide est à la bonne température de traitement, ce qui permet de refroidir très rapidement l'enveloppe. La durée d'efficacité de la séance est de fait optimisée. Grâce à l'invention, la zone du corps est donc globalement mieux traitée qu'avec les machines de l'art antérieur précitées.

D'autres caractéristiques avantageuses de l'invention sont listées ci-dessous. Chacune de ces caractéristiques peut être considérée seule ou en combinaison avec les caractéristiques remarquables définies ci-dessus, et faire l'objet, le cas échéant, d'une ou plusieurs demandes de brevet divisionnaires :
- Selon un mode de réalisation, un système de vannes est agencé dans le circuit hydraulique, de sorte que dans un état de fonctionnement desdites vannes : la quatrième ligne hydraulique est isolée et la troisième ligne hydraulique ouverte, le liquide circulant dans ladite machine, entre les première, deuxième, et troisième lignes hydrauliques.
- Selon un mode de réalisation, un système de vannes est agencé dans le circuit hydraulique, de sorte que dans un état de fonctionnement desdites vannes : la troisième ligne hydraulique est isolée et la quatrième ligne hydraulique ouverte, le liquide circulant, dans ladite machine, dans les première, deuxième, quatrième et cinquième lignes hydrauliques.
- Avantageusement : - le vase d'expansion est à volume réglable et comporte un piston dont la position est réglable pour moduler le volume et/ou la pression du liquide dans le circuit hydraulique ; - le piston est actionné par un vérin, lequel vérin est piloté par une unité de commande en fonction de mesures de pression réalisées au niveau de l'entrée et de la sortie de la pompe.
- Selon un mode de réalisation, la machine comporte en outre l'une des caractéristiques suivantes : - le vase d'expansion est connecté dans une ligne hydraulique reliée à l'entrée de la pompe ; - le vase d'expansion est connecté dans la première ligne hydraulique reliée à la sortie de la pompe ; - un premier vase d'expansion est connecté dans la cinquième ligne hydraulique et un seconde vase d'expansion est connecté dans la première ligne hydraulique.
- Selon un mode de réalisation, la machine comporte : - un dispositif de pressothérapie comportant au moins une pompe alimentant un circuit pneumatique ; - le circuit pneumatique comporte au moins une ligne pneumatique pour connecter la sortie de la pompe pneumatique à au moins une sortie adaptée pour mettre en communication pneumatique ledit circuit pneumatique avec au moins une poche gonflable d'une enveloppe adaptée pour être installée sur une zone à traiter.
- Avantageusement, le circuit pneumatique et le circuit hydraulique sont indépendants l'un de l'autre, ladite machine ayant trois modes de fonctionnement : - un mode de fonctionnement par cryothérapie où seul le circuit hydraulique est utilisé ; - un mode de fonctionnement par pressothérapie où seul le circuit pneumatique est utilisé ; - un mode de fonctionnement combinant la cryothérapie et la pressothérapie et où le circuit hydraulique et le circuit pneumatique sont simultanément est utilisés.
- Avantageusement, le circuit pneumatique comporte au moins une ligne pneumatique pour un traitement par pressothérapie avec effet pompage et/ou au moins une ligne pneumatique pour un traitement par pressothérapie avec effet drainage.
- Selon un mode de réalisation, la machine comporte une platine de connexion, regroupant : - une série de connecteurs adaptés pour connecter des enveloppes au circuit hydraulique ; - une série de connecteurs adaptés pour connecter des enveloppes au circuit pneumatique.
- Selon un mode de réalisation, le circuit hydraulique comporte : - plusieurs sorties reliées à plusieurs branches de la quatrième ligne hydraulique et par lesquelles le liquide refroidi circule depuis ladite quatrième ligne vers plusieurs enveloppes ; - plusieurs entrées reliées à la cinquième ligne hydraulique et par lesquelles le liquide refroidi circule depuis les enveloppes vers ladite cinquième ligne.
- Avantageusement, chaque branche de la quatrième ligne hydraulique est pourvue d'une vanne, lesquelles vannes sont adaptées pour isoler ou ouvrir de manière indépendante les branches dans lesquelles elles sont respectivement installées.
- Un capteur de température peut être connecté dans le circuit hydraulique, en aval de la sortie du réservoir.

Un autre aspect de l'invention concerne un système de traitement par cryothérapie d'une zone d'un corps humain ou animal, le système comportant :
- une machine adaptée pour un traitement par cryothérapie,
- au moins une enveloppe adaptée pour être installée sur la zone à traiter,
- la machine est conforme à l'une des caractéristiques précédentes,
- l'enveloppe est connectée à l'entrée et à la sortie du circuit hydraulique de la machine de sorte que le liquide refroidi circule dans ladite enveloppe.

Encore un autre aspect de l'invention concerne un procédé de fonctionnement d'une machine conforme à l'une des caractéristiques précédentes. Avant une séance de cryothérapie, le procédé comportant les étapes consistant à :
- déterminer une plage cible de températures du liquide, laquelle plage a une borne supérieure et une borne inférieure,
- mesurer la température du liquide dans le circuit hydraulique de la machine,
- comparer la température mesurée à la plage cible de température,
- faire fonctionner la machine selon un mode de fonctionnement qui dépend du résultat de cette comparaison.

Avantageusement, si la température mesurée est égale ou supérieure à la borne supérieure, alors le procédé comporte les étapes consistant à :
- isoler la quatrième ligne hydraulique, ouvrir la troisième ligne hydraulique et actionner la pompe de sorte que le liquide circule en circuit fermé entre les première, deuxième et troisième lignes hydrauliques,
- actionner l'unité de réfrigération de manière refroidir le liquide.

Avantageusement, si la température mesurée est comprise entre la borne inférieure et la borne supérieure, alors le procédé comporte les étapes consistant à désactiver l'unité de réfrigération et désactiver la pompe pour empêcher le refroidissement et la circulation du liquide dans le circuit hydraulique.

Pendant une séance de cryothérapie pour un traitement non thérapeutique, le procédé comporte avantageusement les étapes consistant à :
- isoler la troisième ligne hydraulique, ouvrir la quatrième ligne et actionner la pompe de sorte que le liquide circule en circuit fermé entre la première ligne hydraulique, la deuxième ligne hydraulique, la quatrième ligne hydraulique, l'enveloppe et la cinquième ligne hydraulique,
- déterminer une plage de températures de traitement du liquide, laquelle plage a une borne supérieure et une borne inférieure,
- mesurer la température du liquide dans le circuit hydraulique de la machine,
- comparer la température mesurée à la plage de températures de traitement,
- si la température mesurée est égale ou supérieure à la borne supérieure alors : actionner l'unité de réfrigération de manière à refroidir le liquide,
- si la température mesurée est comprise entre la borne inférieure et la borne supérieure alors : désactiver l'unité de réfrigération pour ne pas refroidir le liquide.

### Description des figures.

D'autres avantages et caractéristiques de l'invention apparaîtront mieux à la lecture de la description d'un mode de réalisation préféré qui va suivre, en référence aux dessins annexés, réalisés à titre d'exemples indicatifs et non limitatifs et sur lesquels :
- la figure 1 schématise une machine conforme à l'invention selon un premier mode de réalisation,
- la figure 2 schématise le fonctionnement de la machine de la figure 1, selon un premier mode de fonctionnement,
- la figure 3 schématise le fonctionnement de la machine de la figure 1, selon un second mode de fonctionnement,
- la figure 4 schématise une machine conforme à l'invention selon un deuxième mode de réalisation,
- la figure 5 schématise une machine conforme à l'invention selon un troisième mode de réalisation,
- la figure 6 schématise une machine conforme à l'invention selon un quatrième mode de réalisation intégrant un dispositif pour un traitement par pressothérapie,
- la figure 7 schématise un dispositif pour un traitement par pressothérapie,
- la figure 8 est un diagramme illustrant une gestion de la température avec une machine conforme à l'invention,
- la figure 9 illustre de manière simplifiée la structure d'une unité de commande utilisée dans l'invention,
- la figure 10 schématise une machine conforme à l'invention selon un cinquième mode de réalisation,
- la figure 11 illustre une platine pour la connexion des enveloppes à la machine M,
- la figure 12 schématise un dispositif pour un traitement par pressothérapie selon une variante de réalisation.

### Modes préférés de réalisation de l'invention.

Selon l'invention, on entend par « A et/ou B » : A ou B ou A+B.

La machine objet de l'invention est principalement adaptée pour un traitement par cryothérapie d'au moins une zone d'un corps humain ou animal. La machine est toutefois également adaptée pour un traitement par pressothérapie d'au moins une zone d'un corps humain ou animal.

La zone du corps à traiter peut être une cheville, un mollet, une cuisse, un poigné, un bras, une épaule, etc. Le traitement peut être à usage thérapeutique (lequel traitement thérapeutique n'est pas couvert par les revendications), en ce sens qu'il contribue à soigner une pathologie, ou à usage non thérapeutique, pour une récupération musculaire par exemple.

La machine comporte avantageusement un châssis, par exemple réalisée en plastique ou à partir d'un assemblage de tôles métalliques. Les différents éléments constitutifs de la machine sont installés à l'intérieur de ce châssis.

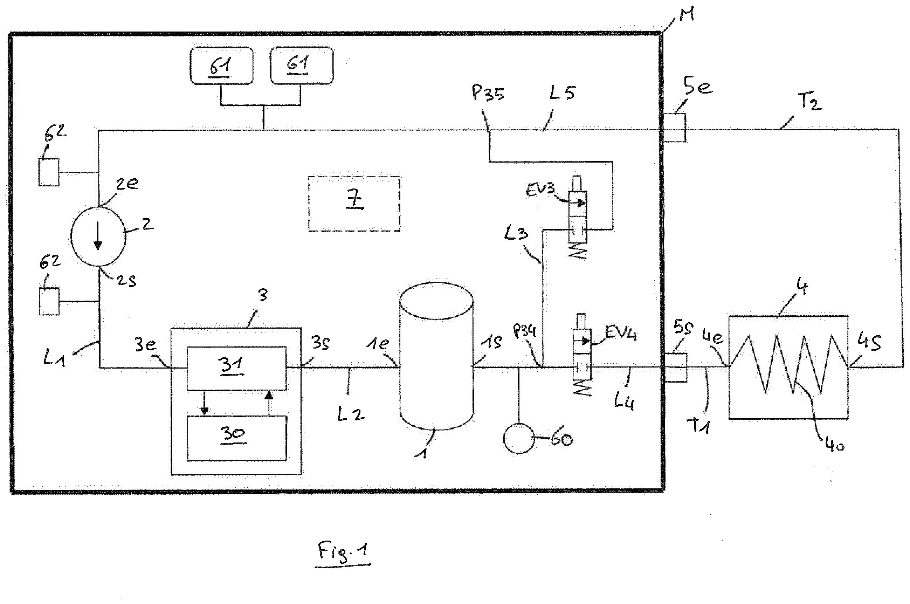

Sur la figure 1, la machine M comporte un réservoir 1 de liquide présentant une entrée 1e et une sortie 1s. Ce réservoir 1 a par exemple une capacité de 10 Litres. Le liquide utilisé est préférentiellement de l'eau déminéralisée mélangée à un antigel (par exemple de l'éthylène glycol ou du propylène glycol). Le pourcentage en volume de l'antigel peut varier de 20% à 50%. D'autres liquides peuvent être utilisés, comme de l'huile par exemple.

La machine M comporte également une pompe hydraulique 2 présentant une entrée d'aspiration du liquide 2e et une sortie de refoulement du liquide 2s. On utilise une pompe hydraulique classique du type pompe à engrenages, pompe à palettes, pompe à pistons, etc. La pompe 2 est dimensionnée de manière à ce que le débit du liquide au niveau de la sortie 2s soit par exemple compris entre 1 L/min et 15 L/min sous une pression comprise entre 0,1 bar à 4 bars. La pompe 2 est la seule pompe hydraulique connectée au circuit hydraulique.

La pompe 2 est située en amont du réservoir 1, c'est-à-dire que par rapport au trajet de circulation du liquide, la sortie 2s de ladite pompe est placée avant l'entrée 1e dudit réservoir.

Le régime de la pompe 2 est contrôlée par une unité de commande 7 en fonction de signaux de pression émis par des capteurs de pression 62 installés dans le circuit hydraulique et connectés de manière filaire ou sans fil à ladite unité de commande. Avantageusement, un capteur de pression 62 est disposé dans la troisième L3 ou cinquième ligne L5, au niveau de l'entrée 2e de la pompe 2, et un autre capteur 62 est installé dans la première ligne L1, au niveau de la sortie 2s de ladite pompe. En fonction des valeurs de pression mesurées par ces capteurs 62, l'unité de commande 7 va modifier le régime de fonctionnement de la pompe 2 pour adapter son débit, notamment en fonction du nombre d'enveloppes connectées à la machine M.

Comme expliqué plus avant dans la description, une ou plusieurs enveloppes peuvent être connectées simultanément à la machine M. On doit donc être capable d'adapter le régime de la pompe 2 en fonction du nombre d'enveloppes. Pour ce faire, on utilise préférentiellement une pompe à gestion électronique ayant des plages de fonctionnement très larges, par exemple de 2 L/min à 10 L/min, pour des pressions de refoulement constante ou quasi-constante.

La machine M comporte encore une unité de réfrigération 3. Elle comporte une entrée 3e et une sortie 3s. Cette unité 3 renferme un groupe frigorifique 30 connecté à un échangeur de chaleur 31. Ce dernier peut être un échangeur à plaques, à tubes, à spirales, etc. Lorsque le liquide pénètre par l'entrée 3s de l'unité 3, il transfert ses calories au fluide frigorigène du groupe frigorifique 30, cet échange étant réalisé au niveau de l'échangeur 31. Le liquide est ainsi refroidi lorsqu'il ressort par la sortie 3s de l'unité 3. Cette unité 3 est avantageusement adaptée pour refroidir le liquide jusqu'à 0°C.

Le réservoir 1, la pompe 2 et l'unité de réfrigération 3 sont connectés au travers d'un circuit hydraulique présentant plusieurs lignes de connexion hydrauliques. Ces lignes sont formées par un ou plusieurs tubes hydrauliques rigides ou flexibles.

Une première ligne hydraulique L1 relie la sortie 2s de la pompe 2 à l'entrée 3e de l'unité de réfrigération 3. Une deuxième ligne hydraulique L2 relie la sortie 3s de l'unité de réfrigération 3 à l'entrée 1s du réservoir 1. Une troisième ligne hydraulique L3 relie la sortie 1s du réservoir 1 à l'entrée 2e de la pompe 2.

Sur la figure 1, la machine M comporte en outre un dispositif de connexion 5e, 5s adapté pour mettre en communication hydraulique le circuit hydraulique avec une enveloppe 4 adaptée pour être installée sur la zone à traiter. Cette enveloppe est du type connue et par exemple conçue selon les enseignements des documents brevets US2002/0138033 (ELKINS), US7198093(ELKINS) ou encore US2010/0145421 (TOMLINSON) auxquels l'homme du métier pourra se référer en cas de besoin. Cette enveloppe 4 peut notamment se présenter sous la forme d'une orthèse adaptée à l'anatomie de la zone du corps à traiter.

L'enveloppe 4 comporte un cheminement 40 à l'intérieur duquel le liquide refroidi issu de la machine M peut circuler. Ce cheminement 40 comporte une entrée 4e et une sortie 4s. L'entrée 4e est connectée à un connecteur de sortie 5s de la machine M, par exemple au moyen d'un tuyau flexible T1. De même, la sortie 4s est connectée à un connecteur d'entrée 5e de la machine M au moyen d'un tuyau flexible T2. La connexion entre les tuyaux T1, T2 et le dispositif de connexion 5e, 5s, est avantageusement réalisée au moyen de raccords démontables du type raccords rapides. L'enveloppe 4 peut ainsi être facilement déconnectée de la machine M. On peut toutefois envisager que l'enveloppe 4 soit connectée de manière permanente à la machine M.

Le circuit hydraulique de la machine M comporte une quatrième ligne hydraulique L4 reliant la sortie 1s du réservoir 1 à la sortie 5s, et une cinquième ligne hydraulique L5 reliant l'entrée 5e à l'entrée 2e de la pompe 2.

Sur la figure 1, la troisième ligne L3 et la cinquième ligne L5 se rejoignent à un point P35 du circuit hydraulique de sorte qu'elles ont une portion de ligne commune entre ce point P35 et l'entrée 2e de la pompe 2. De même, la troisième ligne L3 et la quatrième ligne L4 se rejoignent à un point P34 du circuit hydraulique, de sorte qu'elles ont une portion de ligne commune entre ce point P34 et la sortie 1s du réservoir 1. Cette configuration permet de simplifier la conception du circuit hydraulique et d'utiliser, pour ces portions de lignes communes, des distributeurs (d'alimentation et de retour).

La pompe 2 n'est plus interposée entre la sortie 1s du réservoir 1 et la sortie 5s du circuit hydraulique. La pompe 2 n'est donc plus susceptible de réchauffer le liquide sortant du réservoir 1 et circulant vers l'enveloppe 4 comme enseigné dans les machines de l'art antérieur précité.

En outre, le fait de disposer la pompe 2 directement en amont de l'unité de réfrigération 3 est également avantageux. On réduit au minimum les pertes de charges entre la sortie 2s de la pompe 2 et l'entrée 3e de l'échangeur 31. De fait, on maintient un débit constant dans l'échangeur 31, protégeant ainsi ce dernier contre les risques de givre susceptibles de provoquer son éclatement ou son éventration.

Le contrôle de la température du liquide est notamment réalisé au moyen d'un capteur de température 60. Ce dernier peut être intégré dans le réservoir 1, mais il est avantageusement connecté dans le circuit hydraulique, en aval de la sortie 1s du réservoir 1. On peut ainsi mesurer la température du liquide après le réservoir 1, au plus prêt de la sortie 5s du circuit hydraulique, c'est-à-dire au plus prêts de l'enveloppe 4. Le capteur 60 est plus particulièrement installé entre la sortie 1s du réservoir 1 et le point P34 du circuit hydraulique de manière à pouvoir mesurer la température du fluide qui circule dans la troisième ligne L3 ou dans la quatrième ligne L4. Le capteur 60 est du type connu de l'homme du métier, par exemple une sonde PT100. On peut aussi envisager d'installer un autre capteur de température à l'entrée 2e de la pompe 2, en complément du capteur 60.

Une unité de commande 7 permet de piloter la pompe 2 et l'unité de réfrigération 3 notamment en fonction de la température mesurée par le capteur 60. Ce pilotage est décrit plus avant dans la description. L'unité de commande 7 se présente par exemple sous la forme d'un automate programmable connecté de manière filaire ou sans fils aux différents éléments de la machine M.

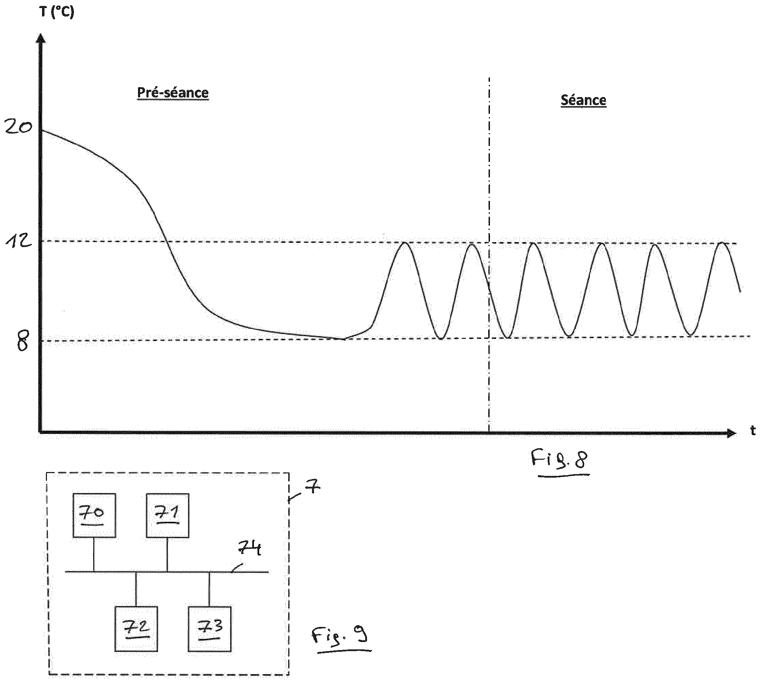

Sur la figure 9, l'unité de commande 7 comprend notamment un ou plusieurs processeurs ou microprocesseurs 70, une ou plusieurs mémoires 71, un module de communication 72, éventuellement une interface réseau 73, qui sont mutuellement connectés via un bus 74. Une ou plusieurs applications informatiques - ou programmes informatiques - sont enregistrées dans la ou les mémoires 71 et dont les instructions (ou codes), lorsqu'elles sont exécutées par le ou les processeurs 70 permettent de réaliser les fonctionnalités de la machine M. Par souci de clarté, il faut comprendre au sens de l'invention que *« la machine M*/*l'unité de commande 7 fait quelque chose* » signifie « *l'application informatique exécutée par le processeur de l'unité de commande 7 fait quelque chose* ». Tout comme « *l'application informatique fait quelque chose* » signifie « *l'application informatique exécutée par le processeur de l'unité de commande 7 fait quelque chose ».*

La ou les mémoires 71 doivent être considérées comme un dispositif de stockage également adapté pour stocker des données et/ou des fichiers de données telles que des précédentes mesures de température et/ou de pression. Il peut s'agir d'une mémoire native ou d'une mémoire rapportée telle qu'une carte Secure Digital (SD).

Le module de communication 72 est adapté pour échanger des signaux radiofréquences transmis sans fil avec un terminal mobile appairé avec la machine M. Dans le but de simplifier la conception, les signaux radiofréquences sont préférentiellement des signaux utilisant un protocole Bluetooth. Toutefois, d'autres protocoles tels que : ISM, Wifi, ANT, ZIGBEE, .., peuvent être utilisés. Le terminal mobile se présente par exemple sous la forme d'un Smartphone (téléphone intelligent) du type iPhone®, Samsung Galaxy®, ou sous la forme d'un autre terminal électronique, par exemple une tablette électronique tactile (du type iPad®, Samsung GalaxyTab®, fonctionnant avec un système d'exploitation de type Windows, Mac, iOS, Android, etc. Ce terminal mobile est adapté pour être exploité par un utilisateur, qui est en pratique, le propriétaire de la machine M. Cet utilisateur peut ainsi commander le fonctionnement de la machine M depuis son terminal mobile. Il est à noter que la commande de la machine M peut se faire de manière alternative ou complémentaire depuis une interface dédiée installée directement sur ladite machine, et qui se présente par exemple sous la forme d'un écran digital tactile.

Le module de communication 72 peut en outre être utilisé pour communiquer avec les différents capteurs et/ou actionneurs de la machine M pour recevoir des données et/ou transmettre des instructions.

L'interface réseau 73 est adaptée pour établir une communication entre la machine M et un serveur informatique distant. L'interface réseau 73 peut par exemple comprendre un module GSM fournissant une connectivité de réseau internet à la machine M. De manière générale, l'interface réseau 73 a pour fonction de gérer les connexions entre la machine M et un réseau internet de sorte que ladite machine puisse être commandée à distance.

Sur la figure 1, un système de vannes EV3, EV4 est installé dans le circuit hydraulique. Une vanne EV3 est installée dans la troisième ligne L3, après la sortie 1s du réservoir 1 et plus particulièrement entre cette sortie 1s et le point P35. Une autre vanne EV4 est installée dans la quatrième ligne L4, après la sortie 1s du réservoir 1 et plus particulièrement entre cette sortie 1s et la sortie 5s du circuit hydraulique. Les vannes EV3, EV4 sont préférentiellement des électrovannes pilotées par l'unité de commande 7. Chaque vanne a une position fermée dans laquelle elle isole la ligne dans laquelle elle est installée (c'est-à-dire qu'elle empêche toute circulation de liquide dans la ligne) et une position ouverte dans laquelle elle autorise une circulation du liquide dans ladite ligne (la ligne est alors ouverte). Sur la figure 1, les vannes EV3 et EV4 sont schématisées dans la position fermée. Pour des raisons de sécurité, la vanne EV3 est naturellement ouverte et la vanne EV4 naturellement fermée.

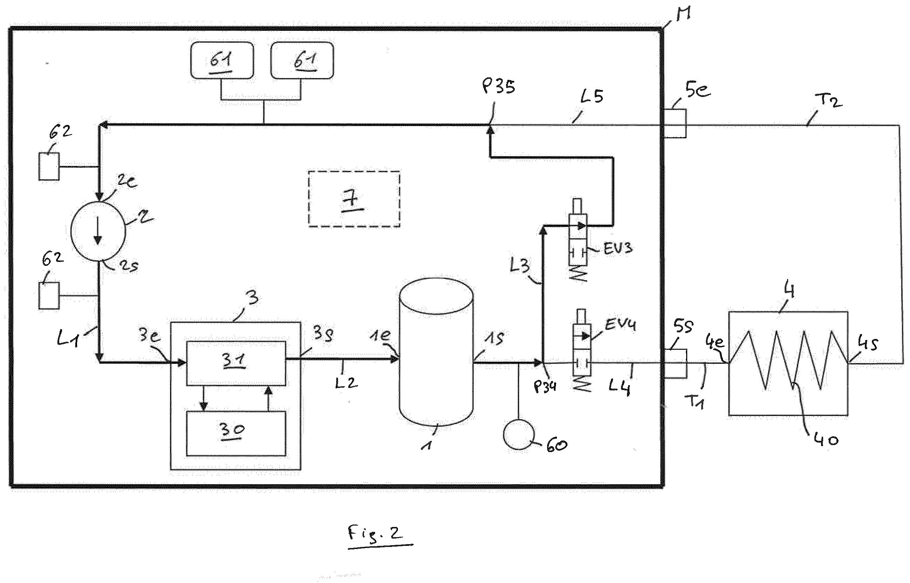

La figure 2 illustre un mode de fonctionnement de la machine M où les vannes EV3 et EV4 sont commandées de sorte que la quatrième ligne L4 soit isolée et la troisième ligne L3 ouverte. Pour ce faire, la vanne EV4 est dans la position fermée et la vanne EV3 est dans la position ouverte. Lorsque la pompe 2 est actionnée, le liquide circule ainsi en circuit fermé, sous pression, dans la machine M, entre les première L1, deuxième L2 et troisième L3 lignes hydrauliques. Le trajet du fluide est schématisé par les flèches en gras. Le liquide pénètre dans la pompe 2 au niveau de son orifice d'aspiration 2e, puis en est refoulé sous pression dans la première ligne L1, au niveau de l'orifice de refoulement 2s. Le liquide sous pression pénètre alors dans l'unité de réfrigération 3 au niveau de son entrée 3e et en ressort refroidi (si le groupe frigorifique 30 est activé) au niveau de sa sortie 3s. Ce liquide refroidi circule ensuite dans la deuxième ligne L2 pour pénétrer dans le réservoir 1 au niveau de son entrée 1e et en ressortir au niveau de sa sortie 1s comme expliqué précédemment.

Dans ce mode de fonctionnement, le circuit hydraulique est un circuit fermé dans lequel le liquide circule sous pression. Cette typologie permet d'éviter les risques d'envasement et d'entartrement, empêchant la prolifération des bactéries.

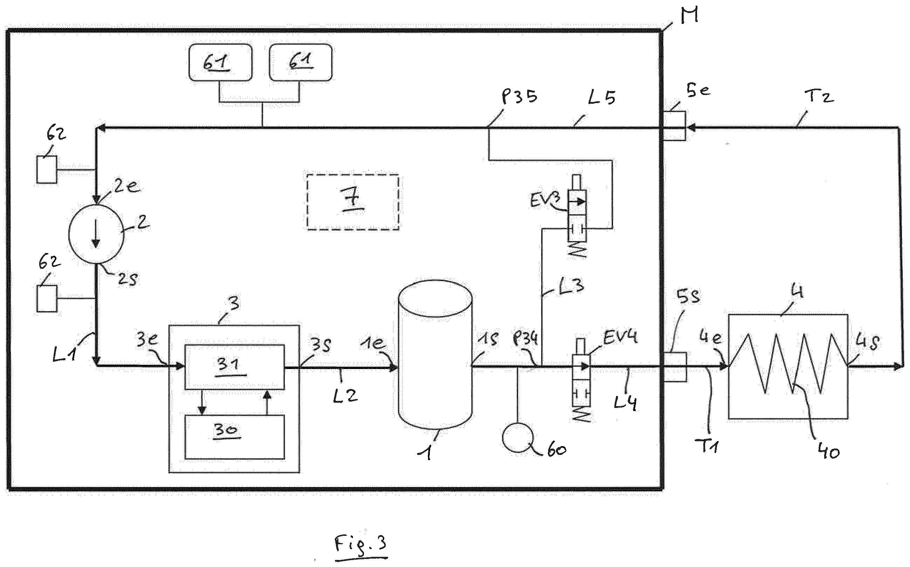

La figure 3 illustre un mode de fonctionnement de la machine M où les vannes EV3 et EV4 sont commandées de sorte que la troisième ligne L3 soit isolée et la quatrième ligne L4 ouverte. Pour ce faire, la vanne EV3 est dans la position fermée et la vanne EV4 est dans la position ouverte. Le trajet du fluide est schématisé par les flèches en gras. Le liquide pénètre dans la pompe 2 au niveau de son orifice d'aspiration 2e, puis en est refoulé sous pression dans la première ligne L1, au niveau de l'orifice de refoulement 2s. Le liquide sous pression pénètre alors dans l'unité de réfrigération 3 au niveau de son entrée 3e et en ressort refroidi (si le groupe frigorifique 30 est activé) au niveau de sa sortie 3s. Ce liquide refroidi circule ensuite dans la deuxième ligne L2 pour pénétrer dans le réservoir 1 au niveau de son entrée 1e et en ressortir au niveau de sa sortie 1s. Le liquide circule ensuite dans la quatrième ligne L4 et sort de la machine M au niveau de la sortie 5s. Le liquide circule dans la tubulure T1, pénètre dans l'enveloppe 4 au niveau de son entrée 4e et en ressort par sa sortie 4s. Le liquide rentre de nouveau dans la machine M, au niveau de l'entrée 5e, via la tubulure T2. Le liquide circule alors dans la cinquième ligne L5 pour être aspiré dans la pompe 2 au niveau de son orifice d'aspiration 2e comme expliqué précédemment.

On constate que dans ce mode de fonctionnement, le liquide circule encore en circuit fermée sous pression dans les première L1, deuxième L2, quatrième L4 et cinquième L5 lignes hydrauliques ainsi que dans les tubulures T1 et T2 et dans l'enveloppe 4.

Dans les modes de fonctionnement des figures 2 et 3, 100% du volume de liquide refoulé par la pompe 2 circule au travers de l'échangeur 31. Le liquide peut donc être refroidi plus rapidement qu'avec les machines décrites dans les documents brevets US2010/0145421 et US2010/0139294 précités. La machine M objet de l'invention permet ainsi un traitement beaucoup plus réactif de la zone à traiter.

Un ou plusieurs débitmètres peuvent être installés dans le circuit hydraulique, notamment entre la pompe 2 et l'entrée 3e de l'unité de réfrigération 3. Ce débitmètre permet de mesurer le débit circulant dans l'échangeur 31. La mesure du débit en direct assure la sécurité de l'échangeur 31. En effet, si le débit circulant dans l'échangeur 31 est inférieur aux préconisations du constructeur, alors il peut givrer et s'éventrer. En fonction de la mesure du débitmètre, l'unité de commande 7 est ainsi capable de couper l'unité de réfrigération 3 et/ou de modifier le régime de la pompe 2 pour ajuster le débit circulant dans l'échangeur 31. De même, si le débit circulant dans l'échangeur 31 est trop élevé, l'unité de commande 7 peut modifier le régime de la pompe 2 pour abaisser ce débit.

A l'intérieur de la machine M, la tuyauterie formant les lignes hydrauliques L1, L2, L3, L4 et L5 a un volume constant. A cause de son refroidissement, la densité du liquide circulant dans cette tuyauterie augmente, laquelle augmentation de densité entraine une augmentation de la résistance de l'écoulement faisant augmenter la pression dans le circuit hydraulique. De plus, l'enveloppe 4 génère une résistance (perte de charge) qui augmente la pression de refoulement de la pompe 2. En outre, l'enveloppe 4 voit son volume varié sous l'effet du liquide sous pression qui circule dans le cheminement 40. Son volume varie proportionnellement à la résistance qu'elle impose au liquide.

Travaillant en circuit fermé, toute modification de volume et de pression sur la ligne de refoulement (ligne entre la sortie 2s de la pompe 2 et la sortie 5s du circuit hydraulique : lignes L1, L2, L4), génère des dépressions sur la ligne d'aspiration (ligne entre l'entrée 5e du circuit hydraulique et l'entrée 2e de la pompe 2 : ligne 5 ; ou ligne entre la sortie 1s du réservoir 1 et l'entrée 2e de la pompe 2 : ligne 3), lesquelles dépressions risquent de faire caviter la pompe 2. Il apparait donc opportun de régler ces contraintes, à savoir compenser les variations de volume et/ou de pression hydraulique dans un circuit hydraulique fermé sous pression, notamment pour éviter toute défaillance de la pompe 2.

Pour ce faire, et comme illustré sur les figures 1 à 6, la ligne d'aspiration est connectée à un ou plusieurs vases d'expansion 61. Ces vases 61 sont adaptés pour compenser les variations de pression et/ou de volume du liquide dans le circuit hydraulique. Cette compensation est avantageusement réalisée dans la cinquième ligne, en amont de l'entrée 2e de la pompe 2, pour préserver au maximum les performances de ladite pompe. Et c'est généralement dans la cinquième ligne L5, après le passage dans l'enveloppe 4, que la pression du liquide est la plus faible. Les vases d'expansion 61 sont préférentiellement du type réservoir à vessie, ayant par exemple une capacité de 5 Litres chacun. Ces vases 61 permettent de faire une réserve de liquide sous pression et jouent le rôle de tampon. Ils comportent généralement une vessie en butyle pourvue d'une valve pour le gonflage. Lorsque la pression du liquide dans la cinquième ligne L5 est inférieure à la pression de réglage des vases d'expansion 61 (qui correspond à la pression cible du liquide dans le réseau hydraulique), ces derniers réinjectent du liquide sous pression. De même, lorsque la pression du liquide dans la cinquième ligne L5 est supérieure à la pression de réglage des vases d'expansion 61, ceux-ci agissent comme un détendeur pour réduire la pression.

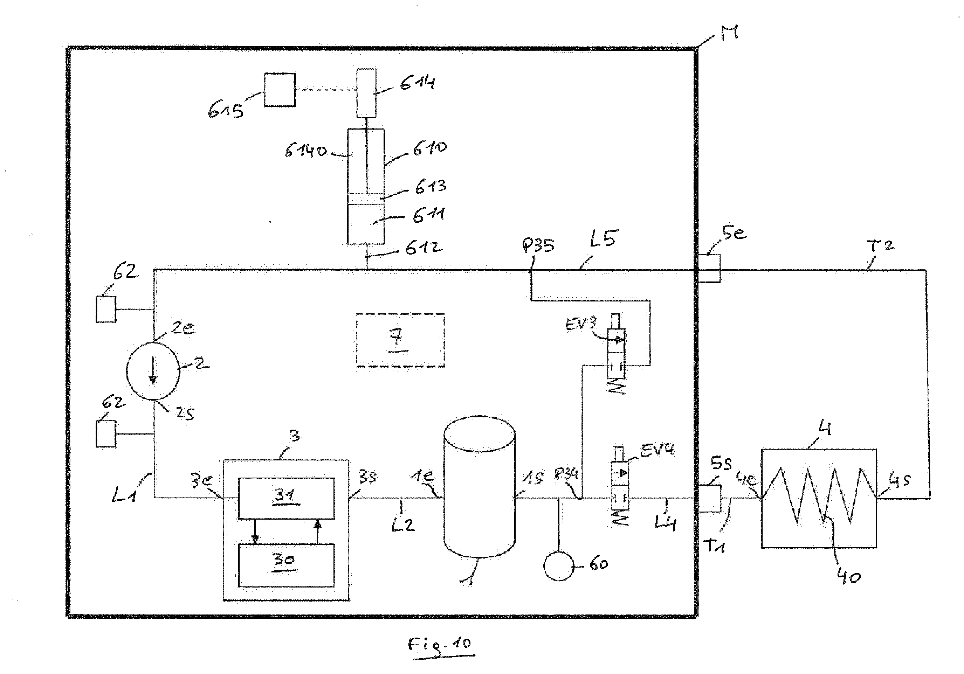

Dans la variante préférée de réalisation de la figure 10, la compensation des variations de pression et/ou de volume du liquide dans le circuit hydraulique est assurée par au moins un vase d'expansion 610 dont le volume est réglable. On peut envisager d'intégrer plusieurs vases d'expansion à volume réglable 610 en cas de besoin.

Le vase 610 comporte une chambre 611 remplie de liquide, laquelle chambre est en communication fluidique avec la ligne d'aspiration L5 au moyen d'une conduite 612. Le volume de la chambre 611 est variable (par exemple entre 0 et 5 Litres) au moyen d'un piston 613 actionné par un vérin 614. La tige 6140 du vérin 614 est avantageusement solidarisée au piston 613 au moyen d'une articulation de type rotule ou pivot de manière à surmonter d'éventuels problèmes de coaxialité entre le piston et l'axe longitudinal de la chambre 611.

L'actionnement du vérin 614 permet de faire coulisser axialement le piston 613 dans la chambre 611 et, de fait, permet de faire varier le volume de ladite chambre pour moduler le volume et/ou la pression du liquide dans le circuit hydraulique. Le vérin 614 est préférentiellement un vérin électrique pour s'assurer d'un contrôle optimal de la position du piston 613 dans la chambre 611. Le vérin 614 est commandé par un contrôleur de position 615 connecté de manière filaire ou sans fil à l'unité de commande 7.

La pression cible du liquide dans le réseau hydraulique est mesurée par les capteurs 62 situés en amont de la pompe 2, au niveau de l'entrée 2e et en aval de ladite pompe, au niveau de la sortie 2s. Ces valeurs sont communiquées à l'unité de commande 7.

Lorsque la pression du liquide dans la ligne d'aspiration L5 est inférieure à la pression cible, l'unité de commande 7 transmet une instruction au contrôleur 615 pour actionner le vérin 614 dans le sens d'une diminution du volume de la chambre 611. Le vase 610 va alors réinjecter du liquide sous pression dans la ligne d'aspiration L5.

De même, lorsque la pression du liquide dans la ligne d'aspiration L5 est supérieure à la pression cible, l'unité de commande 7 transmet une instruction au contrôleur 615 pour actionner le vérin 614 dans le sens d'une augmentation du volume de la chambre 611. Le vase 610 va alors absorber du liquide dans la cinquième ligne L5 et agir comme détendeur pour réduire la pression.

Lorsque le liquide circule dans le circuit hydraulique, la différence de pression entre l'entrée 2e et la sortie 2s de la pompe 2 (ΔP₂) peut varier, selon que des enveloppes 4a, 4b sont connectées ou pas à la machine M.

Lorsque le liquide circule dans la machine M, entre les première L1, deuxième L2, et troisième L3 lignes hydrauliques, sans circuler dans aucune enveloppe 4a, 4b, le ΔP₂ est généralement acceptable, par exemple 0,5 Bars, pour éviter tout problème de cavitation dans la pompe 2.

Lorsque le liquide circule dans la machine M, entre les première L1, deuxième L2, quatrième L4 et cinquième L5 lignes hydrauliques, en circulant dans une ou plusieurs enveloppes 4a, 4b, les pertes de charges augmentent dans le circuit hydraulique. La pression de refoulement de la pompe 2 augmente et sa pression d'aspiration diminue. Il en résulte que le ΔP₂ peut fortement augmenter, pour par exemple atteindre 1,5 Bars. Dans ces conditions, les risques de cavitation dans la pompe 2 sont élevés.

Pour remédier à cela, le vase d'expansion à volume réglable contrôlé 610 est placé dans la ligne d'aspiration L5. Le vérin 614 est alors actionné dans le sens d'une diminution du volume de la chambre 611 pour injecter du liquide sous pression dans la ligne d'aspiration L5. La pression d'aspiration de la pompe 2 va ainsi augmenter de sorte que le ΔP₂ va diminuer jusqu'à atteindre une valeur acceptable.

Selon une variante de réalisation, on peut également envisager de placer le vase d'expansion à volume réglable contrôlé 610 dans la ligne de refoulement L1. Le vérin 614 est alors actionné dans le sens d'une augmentation du volume de la chambre 611 pour absorber du liquide dans la ligne de refoulement L1. La pression de refoulement de la pompe 2 va ainsi diminuer de sorte que ΔP₂ va également diminuer jusqu'à atteindre une valeur acceptable.

Selon encore une autre variante de réalisation, on place un premier vase 610 dans la ligne d'aspiration L5 et un seconde vase 610 dans la ligne de refoulement L1. Le vérin 614 du premier vase est actionné dans le sens d'une diminution du volume de la chambre 611 pour injecter du liquide sous pression dans la ligne d'aspiration L5. Et le vérin 614 du second vase est actionné le sens d'une augmentation du volume de la chambre 611 pour absorber du liquide dans la ligne de refoulement L1. La pression d'aspiration de la pompe 2 va ainsi augmenter et la pression de refoulement diminuer. Cette technique permet d'ajuster plus rapidement le ΔP₂ à une valeur acceptable.

Quel que soit le mode de réalisation, le contrôle du régime de la pompe 2 et le contrôle de l'actionnement du vérin 614, agissent en synergie pour obtenir une plage de fonctionnement plus grande de la pompe 2, comparé à une solution utilisant un vase d'expansion 61 classique.

L'utilisation d'un vase d'expansion à volume réglable contrôlé 610 présente de multiples avantages par rapport à un vase d'expansion classique 61. On supprime la membrane en butyle qui devient poreuse dans le temps. On supprime également la valve de gonflage qui peut fuir. On a une plage de fonctionnement en termes de compensation de pression et/ou de volume qui est beaucoup plus grande. En outre, le contrôle du réglage du volume permet d'avoir une compensation beaucoup plus fine du volume de liquide circulant dans le circuit hydraulique, notamment en cas de perte ou de fuite de liquide au niveau de l'enveloppe 4.

Selon des modes de réalisation non couverts par la présente invention, le vase d'expansion à volume réglable contrôlé 610 est utilisé dans n'importe quel autre circuit hydraulique fermé sous pression, par exemple le circuit hydraulique d'une machine de cryothérapie ayant une conception autre que celle revendiquée, le circuit hydraulique d'une chambre froide, d'un réseau de climatisation, d'une machine frigorifique, etc.

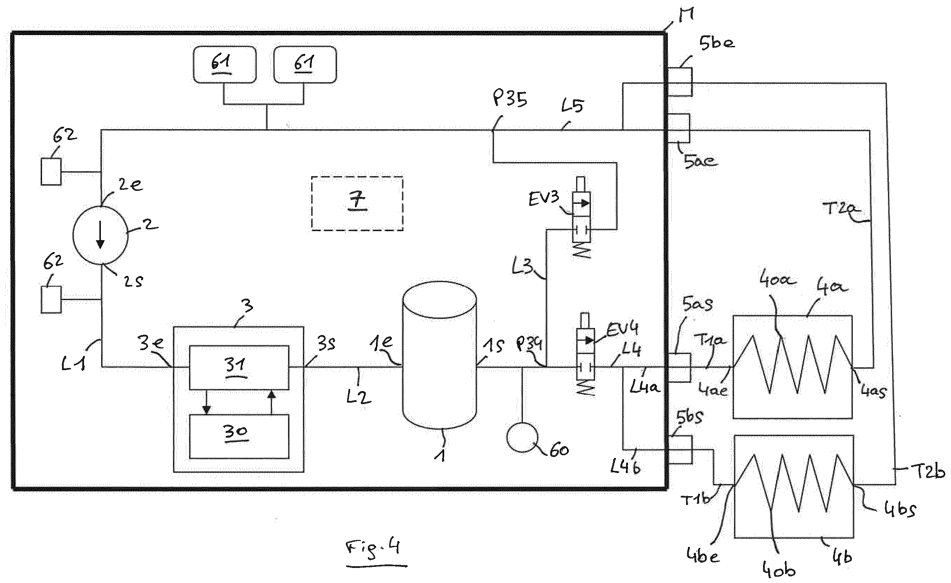

La figure 4 illustre un autre mode de réalisation de la machine M où deux enveloppes 4a, 4b sont connectées simultanément à ladite machine. Ces deux enveloppes sont destinées à être installées sur deux zones distinctes du corps d'une même personne (humaine ou animale). Par exemple, une enveloppe 4a est installée sur un poigné et l'autre enveloppe 4b sur une cuisse.

Le circuit hydraulique de la machine M est ici pourvu de plusieurs entrées 5ae, 5be et plusieurs sorties 5as, 5bs. Comme expliqué précédemment, chaque enveloppe 4a, 4b comporte un ou plusieurs cheminements 40a, 40b, une entrée 4ae, 4be et une sortie 4as, 4bs. Les entrées 4ae, 4be sont connectées respectivement à un connecteur de sortie 5as, 5bs de la machine M, par exemple au moyen d'un tuyau flexible, respectivement T1a, T1b. De même, les sorties 4as, 4bs sont connectées respectivement à un connecteur d'entrée 5ae, 5be de la machine M au moyen d'un tuyau flexible, respectivement T2a, T2b. Les enveloppes 4a et 4b sont connectées de manière permanente ou démontable à la machine M.

La quatrième ligne L4 se divise en deux branches L4a, L4b pour relier simultanément la sortie 1s du réservoir 1 aux sorties 5as, 5bs. La cinquième ligne L5 relie simultanément les entrées 5ae, 5be à l'entrée 2e de la pompe 2. En actionnant la pompe 2, lorsque la vanne EV4 est ouverte et la vanne EV3 fermée, le liquide circule simultanément dans les deux enveloppes 4a et 4b. Le trajet de circulation du liquide est similaire à celui décrit en référence à la figure 3. On peut ainsi traiter simultanément plusieurs zones du corps d'une même personne, avec une seule machine M et une seule pompe 2. Le nombre d'entrées 5ae, 5be, le nombre de sortie 5as, 5bs, et le nombre de branches L4a, L4b de la quatrième ligne L4, seront adaptés au nombre d'enveloppes utilisées.

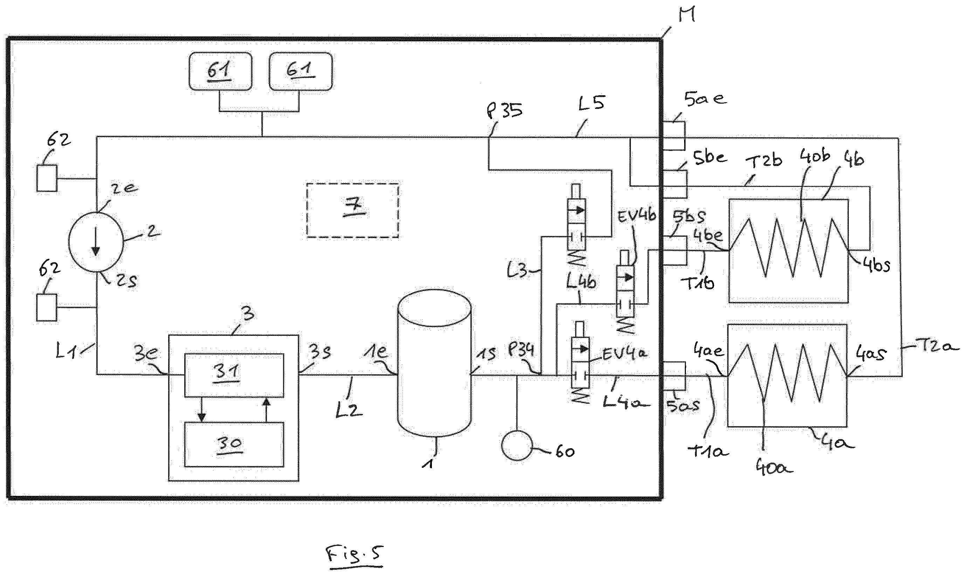

La figure 5 illustre un autre mode de réalisation de la machine M où les deux enveloppes 4a, 4b sont connectées à ladite machine. Ces deux enveloppes sont destinées à être installées sur des zones du corps de deux personnes distinctes (humaines ou animales). Par exemple, l'enveloppe 4a est installée sur le poigné d'une première personne et l'autre enveloppe 4b sur la cuisse d'une deuxième personne.

La configuration de cette machine M est similaire à celle de la figure 4. La quatrième ligne L4 se divise en deux branches L4a, L4b pour relier simultanément la sortie 1s du réservoir 1 aux sorties 5as, 5bs. La cinquième ligne L5 relie simultanément les entrées 5ae, 5be à l'entrée 2e de la pompe 2.

Chacune des branches L4a et L4b de la quatrième ligne L4 est ici pourvue d'une vanne, respectivement EV4a et EV4b. Ces vannes sont identiques à celles décrites précédemment. La vanne EV4a est installée entre la sortie 1s du réservoir 1 et la sortie 5as du circuit hydraulique. Et la vanne EV4b est installée entre la sortie 1s du réservoir 1 et la sortie 5bs du circuit hydraulique. Ces vannes EV4a et EV4b permettent d'isoler ou d'ouvrir de manière indépendante les branches L4a et L4b selon le nombre de personnes à traiter. Par exemple, si une seule personne doit être traitée, seule l'enveloppe 4a est destinée à être utilisée. La vanne EV4b sera alors maintenue en position fermée pour isoler la branche L4b et la vanne EV4a sera mise en position ouverte pour ouvrir la branche L4a et alimenter l'enveloppe 4a. Si les deux personnes sont traitées simultanément, les vannes EV4a et EV4b seront mises en position ouverte pour ouvrir chacune des branches L4a et L4b et alimenter simultanément les enveloppes 4a et 4b. Le trajet de circulation du liquide est similaire à celui décrit en référence à la figure 3. On peut ainsi traiter simultanément plusieurs personnes, avec une seule machine M et une seule pompe 2. Bien évidemment, il est possible de connecter davantage d'enveloppes (par exemple jusqu'à quatre ou cinq) à la machine M. Auquel cas, le nombre d'entrées 5ae, 5be, le nombre de sortie 5as, 5bs, le nombre de branches L4a, L4b de la quatrième ligne L4 et le nombre de vannes EV4a, Ev4b, seront adaptés au nombre de personnes à traiter. On peut également combiner les configurations des figures 4 et 5 pour traiter simultanément plusieurs personnes, au niveau de plusieurs zones de leur corps, avec une seule machine M et une seule pompe 2.

Le procédé de fonctionnement de la machine M va maintenant être décrit en détail en se rapportant aux figures 2 et 3. Ce fonctionnement est toutefois identique avec les configurations des figures 4 et 5.

Avant de commencer une séance de cryothérapie (pré-séance), nous avons vu qu'il était particulièrement avantageux de porter le liquide à la bonne température de façon à optimiser la durée d'efficacité de la séance.

L'utilisateur commence par déterminer une plage cible de températures du liquide. Cette plage de température a une borne inférieure et une borne supérieure. Les valeurs des bornes supérieure et inférieure sont transmises à l'unité de commande 7 au moyen d'une interface dédiée installée directement sur la machine M, ou à distance, depuis un terminal mobile de l'utilisateur, comme expliqué précédemment. Cette plage cible de températures peut également être préenregistrée dans la mémoire 71 de l'unité de commande 7.

L'unité de commande 7 va relever la température du liquide dans le circuit hydraulique. Ce relevé peut être effectué directement dans le réservoir 1 si ce dernier intègre un capteur de température ou au moyen du capteur 60. Le contrôle de la température du liquide dans le circuit est réalisée de manière continue ou à intervalles de temps.

L'unité de commande 7 va ensuite comparer la température mesurée à la plage cible de températures. Le mode de fonctionnement de la machine M dépend du résultat de cette comparaison.

Si la température mesurée est égale ou supérieure à la borne supérieure, alors l'unité de commande 7 va faire fonctionner la machine M pour refroidir le liquide. Pour ce faire, elle va configurer le circuit hydraulique selon la figure 2 de sorte que la quatrième ligne L4 soit isolée et la troisième ligne L3 soit ouverte. Après avoir actionné la pompe 2, le liquide circule en circuit fermé entre les première L1, deuxième L2 et troisième L3 lignes hydrauliques. L'unité de commande 7 va simultanément actionner l'unité de réfrigération 3 pour refroidir le liquide.

Si la température mesurée est comprise entre la borne inférieure et la borne supérieure, l'unité de commande 7 va faire fonctionner la machine M pour empêcher le refroidissement et la circulation du liquide dans le circuit hydraulique. Elle va ici configurer le circuit hydraulique de sorte que la quatrième ligne L4 soit isolée et la troisième ligne L3 soit ouverte. Toutefois, l'unité de réfrigération 3 va être désactivée pour ne pas refroidir le liquide. La pompe 2 va être également être désactivée pour ne pas faire circuler le liquide. Le liquide contenu dans le réservoir 1 est à la bonne température et la machine M opérationnelle pour une séance de cryothérapie.

Pour initier une séance de cryothérapie, l'utilisateur détermine une plage de températures de traitement du liquide, laquelle plage a une borne supérieure et une borne inférieure. Cette plage de températures de traitement du liquide peut correspondre à la plage cible précitée ou à une autre plage de températures. L'utilisateur peut, le cas échéant, déterminer la durée de la séance, par exemple 20 minutes. Les valeurs des bornes supérieure et inférieure de la plage de températures de traitement, et la durée de la séance, sont transmises à l'unité de commande 7 au moyen d'une interface dédiée installée directement sur la machine M, ou à distance, depuis un terminal mobile de l'utilisateur, comme expliqué précédemment. Différentes plages de températures de traitement et différentes durée de séance peuvent être préprogrammées dans la mémoire 71 de l'unité de commande 7. Dans ce cas, l'utilisateur n'a qu'à sélectionner le programme qu'il souhaite.

L'unité de commande 7 configure le circuit hydraulique selon la figure 3 de sorte que la troisième ligne L3 soit isolée et la quatrième ligne L4 soit ouverte. Après avoir actionné la pompe 2, le liquide circule en circuit fermé entre la première ligne L1, la deuxième ligne L2, la quatrième ligne L4, l'enveloppe 4 et la cinquième ligne L5. Le liquide déjà refroidi lors de la phase pré-séance, va ainsi circuler dans l'enveloppe 4 pour traiter la zone du corps.

Un échange de calories se fait au niveau de l'enveloppe 4, le liquide circulant dans ladite enveloppe étant réchauffé par la chaleur du corps au niveau de la zone traitée. Un échange de calories se fait également au niveau de la pompe 2 qui échauffe le fluide.

Comme pour la phase de pré-séance, l'unité de commande 7 va relever de manière continue ou à intervalles de temps, la température du liquide dans le circuit hydraulique. La température mesurée va être comparée à la plage de températures de traitement.

Si la température mesurée est égale ou supérieure à la borne supérieure, alors l'unité de commande 7 va actionner l'unité de réfrigération 3 de manière refroidir le liquide. Par contre, si la température mesurée est comprise entre la borne inférieure et la borne supérieure, alors l'unité de commande 7 va désactiver l'unité de réfrigération 3 pour ne pas refroidir le liquide. Il y a donc une régulation de la température du liquide tout au long de la séance.

La figure 8 est un diagramme illustrant cette régulation de la température avant une séance de cryothérapie (pré-séance) et pendant la séance. Les ordonnées correspondent à la température du liquide en degrés Celsius et les abscisses au temps.

Avant la séance, la plage cible de températures du liquide est 8°C-12°C. L'unité de commande 7 relève que la température du liquide dans le circuit hydraulique est de 20°C, soit supérieure à la borne supérieure 12°C. L'unité de commande 7 va alors faire fonctionner la machine M pour que le liquide soit refroidi à 8°C. Dès que cette température est atteinte, l'unité de réfrigération 3 et la pompe 2 sont désactivées. Si la température du liquide remonte et devient égale ou supérieure à 12°C, l'unité de commande 7va à nouveau faire fonctionner la machine M pour que le liquide soit refroidi à 8°C. Il y a donc une régulation de la température du liquide dans la plage cible de températures durant la pré-séance.

Au moment d'initier la séance, l'utilisateur défini la plage 8°C-12°C comme plage de traitement. L'unité de commande 7 relève que la température du liquide dans le circuit hydraulique est de 10°C, soit supérieure à la borne inférieure 8°C. L'unité de commande 7 va alors faire fonctionner la machine M, en activant la pompe 2 mais sans activer l'unité de réfrigération 3. Le liquide va se réchauffer (dans l'enveloppe et au passage dans la pompe 2) jusqu'à atteindre 12°C. Dès que la température atteint 12°C, l'unité de commande 7 active l'unité de réfrigération 3 pour refroidir le liquide jusqu'à 8°C. Dès que cette température de 8°C est atteinte, l'unité de commande 7 désactive l'unité de réfrigération 3. Cette régulation va se poursuivre pendant toute la séance.

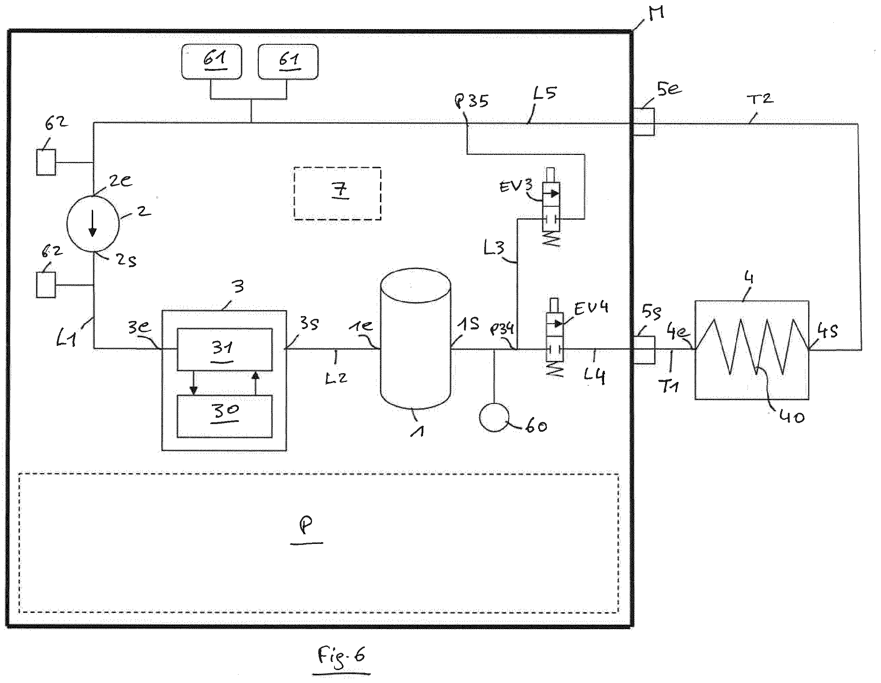

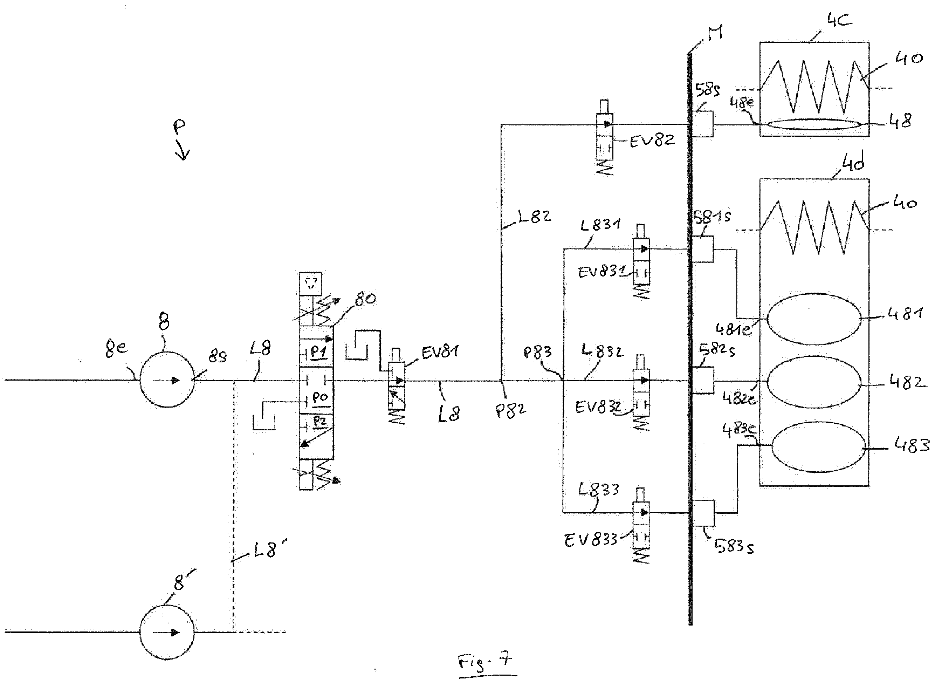

Les figures 6 et 7 illustrent une variante de réalisation où la machine M est également adaptée pour un traitement par pressothérapie. Elle intègre à cet effet un dispositif de pressothérapie P. Ce type de traitement est généralement utilisé pour augmenter la circulation sanguine au niveau de la zone traitée du corps. Dans certains cas, il est bénéfique de combiner un traitement par pressothérapie à un traitement par cryothérapie.

Le dispositif de pressothérapie P est illustré plus en détail sur la figure 7. Il comporte ici une pompe pneumatique 8 présentant une entrée d'aspiration d'air 8e et une sortie de refoulement d'air 8s. On peut utiliser une soufflante, ou une pompe du type, centrifuge, à palettes, à vis, à pistons, etc. La pompe 8 est avantageusement dimensionnée de manière à ce que le débit d'air au niveau de la sortie 8s soit compris entre 15 L/min et 100 L/min sous une pression comprise entre 0,05 bar et 1 bar. La pompe 8 est commandée par l'unité de commande 7.

Un circuit pneumatique permet de connecter la pompe 8 à une ou plusieurs enveloppes 4c, 4d. Ce circuit pneumatique est indépendant du circuit hydraulique précité. Les enveloppes 4c, 4d présentent chacune au moins une poche souple gonflable.

Sur la figure 7, l'enveloppe 4c présente une seule poche gonflable 48 comportant une entrée 48e. La machine M comporte une sortie 58s, adaptée pour mettre en communication le circuit pneumatique avec l'enveloppe 4c et plus particulièrement avec sa poche 48. Ce type d'enveloppe 4c assure un effet de pompage sur la zone à traiter comme expliqué plus avant dans la description. La machine M peut être conçue pour faire fonctionner plusieurs enveloppes 4c, auquel cas ladite machine comporte plusieurs sorties 58s.

Sur la figure 7, l'autre enveloppe 4d présente plusieurs poches gonflables indépendantes 481, 482, 483 (ou une poche segmentée en plusieurs compartiments indépendants). Le nombre de poche peut par exemple varier de 2 à 10 selon la taille de l'enveloppe 4d et/ou la zone à traiter. Ce type d'enveloppe 4d assure un effet de drainage sur la zone à traiter comme expliqué plus avant dans la description. Chaque poche 481, 482, 483 comporte une entrée 481e, 482e, 483e. La machine M comporte plusieurs sorties 581s, 582s, 583s adaptées pour mettre en communication le circuit pneumatique avec l'enveloppe 4d et plus particulièrement avec chacune des poches 481, 482, 483. La machine M peut être conçue pour faire fonctionner plusieurs enveloppes 4d, auquel cas ladite machine comporte plusieurs série de sorties 581s, 582s, 583s.

Les entrées 48e, 481e, 482e, 483e des poches 48, 481, 482, 483 sont connectées aux sorties respectives 58s, 581s, 582s, 583s par exemple au moyen d'un tuyau flexible. Ces connexions sont avantageusement réalisées au moyen de raccords démontables du type raccords rapides. Les poches 48, 481, 482, 483 peuvent ainsi être facilement déconnectées de la machine M. On peut toutefois envisager que les poches 48, 481, 482, 483 soient connectées de manière permanente à la machine M.

Sur la figure 7, les enveloppes 4c, 4d comportent également un cheminement 40 à l'intérieur duquel le liquide refroidi issu de la machine M peut circuler de manière à pouvoir combiner simultanément un traitement par cryothérapie et un traitement par pressothérapie. La connexion du cheminement 40 au circuit hydraulique de la machine M a déjà été décrite précédemment. Les enveloppes 4c, 4d peuvent toutefois être dépourvues d'un tel cheminement dans le cas où elles sont seulement dévolues à un traitement par pressothérapie. La machine M peut donc être connectée à une ou plusieurs enveloppes ne comportant que des cheminements 40 (cryothérapie seule) et/ou à une ou plusieurs enveloppes comportant des cheminements 40 et des poches gonflables (cryothérapie + pressothérapie) et/ou à une ou plusieurs enveloppes ne comportant que des poches gonflables (pressothérapie seule : drainage et/ou pompage).

Un régulateur de pression 80 est installé dans le circuit pneumatique, en aval de la sortie 8s de la pompe 8. Ce régulateur 80 est avantageusement un régulateur électronique de pression connecté à l'unité de commande 7. Il a pour fonction de gérer la pression dans le circuit pneumatique, et de fait la pression dans les poches des enveloppes 4c, 4d. Il se présente par exemple sous la forme d'une électrovanne 3/3 (3 orifices / 3 positions). Dans une position neutre P0, le régulateur 80 interdit toute circulation d'air dans le circuit pneumatique. Dans une position active P1, le régulateur 80 autorise une circulation d'air dans le circuit pneumatique. Et dans une position de purge P2, le régulateur 80 met à l'échappement le circuit pneumatique.

Le régulateur électronique 80 permet de gérer des faibles pressions (jusqu'à 0,1 mmHg). On peut ainsi obtenir un traitement par pressothérapie très sensible, par exemple pour faire du drainage lymphatique où de faibles pressions doivent être exercées sur la zone du corps.

La consigne de régulation de pression est transmise à l'unité de commande 7 par l'utilisateur au moyen d'une interface dédiée installée directement sur la machine M, ou à distance, depuis un terminal mobile de l'utilisateur, comme expliqué précédemment. Différentes consignes de régulation de pression peuvent être préprogrammées dans la mémoire 71 de l'unité de commande 7. Dans ce cas, l'utilisateur n'a qu'à sélectionner le programme qu'il souhaite.

Sur la figure 7, le circuit pneumatique comporte :
- une ligne pneumatique L8 connectée à la sortie du régulateur 80 ;
- une ligne pneumatique L82 reliant la ligne pneumatique L8 à la sortie 58s de la machine M,
- des lignes pneumatiques L831, L832, L833 reliant respectivement la ligne pneumatique L8 aux sorties 581s, 582s, 583s de la machine M.

Cet agencement est donné à titre d'exemple illustratif. Aussi, un autre agencement de lignes pneumatiques peut être envisagé.

Ces lignes pneumatiques sont formées par un ou plusieurs tubes pneumatiques rigides ou flexibles.

Sur la figure 7, la ligne L8 et la ligne L82 se rejoignent à un point commun P82 du circuit pneumatique. De même, la ligne L8 et les lignes L831, L832, L833 se rejoignent à un point commun P83. Cette configuration permet de simplifier la conception du circuit pneumatique et d'utiliser des distributeurs (d'alimentation et de retour). Les points P82 et P83 peuvent consister en un seul et même point.

Les lignes L8 et L82 forment ensemble une ligne pour un traitement par pressothérapie avec effet pompage. Et les lignes L8, L831, L832 et L833 forment ensemble une ligne pour un traitement par pressothérapie avec effet drainage.

Sur la figure 7, un système de vannes EV81, EV82, EV831, EV832, EV833 est installé dans le circuit pneumatique.

La vanne EV81 est installée dans la ligne L8, après la sortie du régulateur 80 et plus particulièrement entre cette sortie et le point P82. Cette vanne EV81 est un moyen de purge des poches 48, 481, 482, 483.

La vanne EV82 est installée dans la ligne L82, entre la sortie de la vanne EV81 et la sortie 58s de la machine M et plus particulièrement entre le point P82 et la sortie 58s.

La vanne EV831 est installée dans la ligne L831, entre la sortie de la vanne EV81 et la sortie 581s de la machine M et plus particulièrement entre le point P83 et la sortie 581s.

La vanne EV832 est installée dans la ligne L832, entre la sortie de la vanne EV81 et la sortie 582s de la machine M et plus particulièrement entre le point P83 et la sortie 582s.

La vanne EV833 est installée dans la ligne L833, entre la sortie de la vanne EV81 et la sortie 583s de la machine M et plus particulièrement entre le point P83 et la sortie 583s.

Les vannes EV81, EV82, EV831, EV832, EV833 sont préférentiellement des électrovannes pilotées par l'unité de commande 7.

La vanne EV81 a une position ouverte dans laquelle elle autorise une circulation d'air dans la ligne L8 (la ligne est alors ouverte) et une position de purge où elle met à l'échappement la ligne L8. Sur la figure 7, la vanne EV81 est schématisée dans la position ouverte.

Chaque vanne EV82, EV831, EV832, EV833 a une position fermée dans laquelle elle isole la ligne dans laquelle elle est installée (c'est-à-dire qu'elle empêche toute circulation d'air dans la ligne) et une position ouverte dans laquelle elle autorise une circulation d'air dans ladite ligne (la ligne est alors ouverte). Sur la figure 7, les vannes EV82, EV831, EV832, EV833 sont schématisées dans la position ouverte.

La vanne EV82 peut être remplacée par un coupleur pneumatique : lorsque le tuyau relié à l'entrée 48e est connecté à la sortie 58s, une circulation d'air est possible depuis la ligne L82 jusque dans la poche 48 ; et lorsque ce tuyau est déconnecté de la sortie 58s, la circulation d'air est automatiquement coupée.

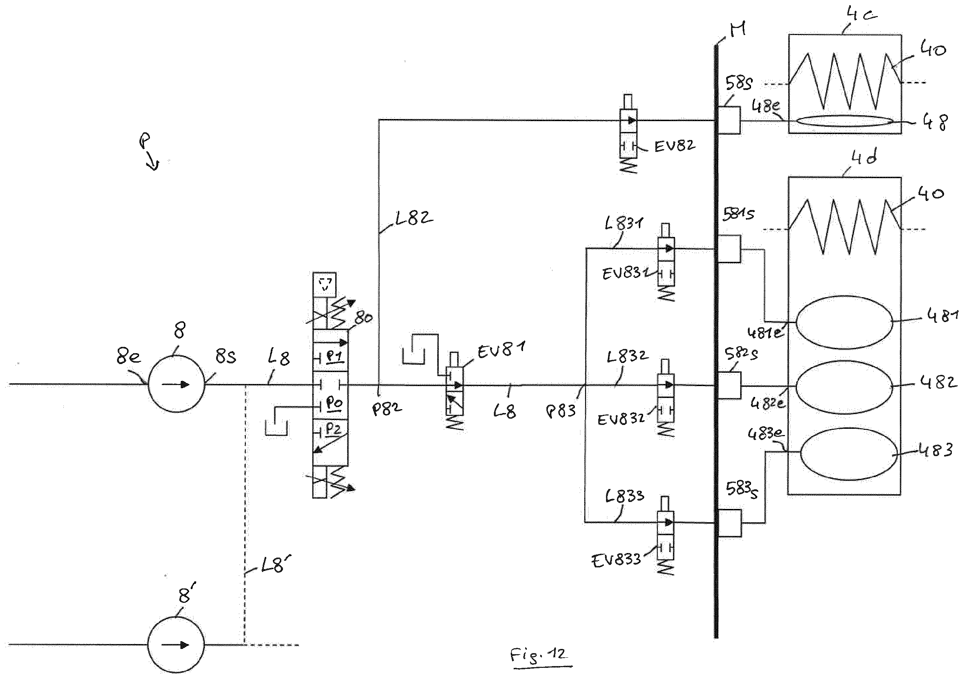

La figure 12 illustre une variante de réalisation où la vanne EV81 est installée dans la ligne L8, après le point P82. La vanne EV81 sert alors à purger uniquement les poches 481, 482, 483. La poche 48 est quant à elle purger au travers du régulateur 80.

Comme indiqué précédemment, l'enveloppe 4c assure un effet de pompage sur la zone à traiter, tandis que l'enveloppe 4d assure un effet de drainage. Le dispositif P assure ces deux types de traitement par pressothérapie. Le dispositif de pressothérapie P peut être conçu uniquement pour gonfler/dégonfler une ou plusieurs enveloppes 4c (pressothérapie avec seulement effet pompage), ou uniquement pour gonfler/dégonfler une ou plusieurs enveloppes 4d (pressothérapie avec seulement effet drainage), ou pour gonfler/dégonfler une ou plusieurs enveloppes 4c et une ou plusieurs enveloppes 4d (pressothérapie avec effet drainage et effet pompage).

Le fonctionnement du dispositif P pour ces traitements va maintenant être décrit plus en détail.

### Effet de pompage

La consigne pour ce type traitement est transmise à l'unité de commande 7 par l'utilisateur au moyen d'une interface dédiée installée directement sur la machine M, ou à distance, depuis un terminal mobile de l'utilisateur, comme expliqué précédemment. Cette consigne de traitement peut être transmise simultanément à la consigne de pression.

Dans le mode de réalisation de la figure 7, l'unité de commande 7 ouvre les vannes EV81 et EV82 et, le cas échéant ferme les vannes EV831, EV832, EV833. Lorsque la pompe 8 est activée, l'air ambiant est aspiré au niveau de l'entrée 8e puis refoulé dans la ligne L8 par la sortie 8s. L'air circule dans la ligne L82 et sort de la machine M au niveau de la sortie 58s. L'air pénètre dans l'enveloppe 4c au niveau de l'entrée 48e. Tant que la pression dans la poche 48, et donc la pression dans la ligne L82, n'atteint pas la pression de consigne, l'air continue de pénétrer dans ladite poche. Dès que la pression de consigne est atteinte, le régulateur 80 régule la pression dans la ligne L82. Pour dégonfler la poche 48, l'unité de commande 7 pilote la vanne EV81 pour la mettre en position de purge où elle met à l'échappement la ligne L82 pour mettre à l'échappement ladite poche. Pour obtenir un effet de pompage, l'unité de commande 7 alterne le gonflage et le dégonflage de la poche 48 (par exemple alternance comprise entre 1 s et 30 s suivant par exemple une courbe de type sinusoïdale). A la fin du traitement, l'unité de commande 7 pilote la vanne EV81 pour mettre à l'échappement la poche 48.

Dans le mode de réalisation de la figure 12, l'unité de commande 7 ouvre simplement la vanne EV82. Le gonflage de la poche 48 et la régulation de sa pression sont identiques à ceux décrits au paragraphe précédent. Pour dégonfler la poche 48, l'unité de commande 7 pilote le régulateur 80 (pression de consigne nulle) pour mettre à l'échappement ladite poche. C'est donc le régulateur qui assure le dégonflage de la poche 48.

### Effet de drainage

L'effet drainage consiste à gonfler/dégonfler les poches 481, 482, 483 pour obtenir un effet de « vague » sur la zone à traiter.

On peut par exemple gonfler la première poche 481, puis la deuxième poche 482 (en maintenant la première poche gonflée), puis la troisième poche 483 (en maintenant la première poche et la deuxième poche gonflées). On peut ensuite dégonfler la troisième poche 483 (en maintenant la première poche et la deuxième poche gonflées), puis dégonfler la deuxième poche 482 (en maintenant la première poche gonflée et la troisième poche dégonflée), puis éventuellement dégonfler la première poche 481 (en maintenant la deuxième poche dégonflée et la troisième poche dégonflée). Et ainsi de suite pour obtenir un effet de vague qui monte et descend le long de tout ou partie de la zone à traiter.

On peut encore par exemple gonfler la première poche 481, puis la dégonfler, puis gonfler la deuxième poche 482 (en maintenant la première poche et la troisième poche dégonflées) et la dégonfler, puis gonfler la troisième poche 483 (en maintenant la première poche et la deuxième poche dégonflées) et la dégonfler. Et ainsi de suite pour obtenir un effet de vague qui monte et descend le long de tout ou partie de la zone à traiter.

La consigne pour ce type traitement est transmise à l'unité de commande 7 par l'utilisateur au moyen d'une interface dédiée installée directement sur la machine M, ou à distance, depuis un terminal mobile de l'utilisateur, comme expliqué précédemment. Cette consigne de traitement peut être transmise simultanément à la consigne de pression.

On va décrire un exemple de traitement par drainage consistant dans une première phase à gonfler la première poche 481, puis la deuxième poche 482, puis la troisième poche 483, et consistant, dans une seconde phase à dégonfler la troisième poche 483, puis dégonfler la deuxième poche 482, puis dégonfler la première poche 481.

L'unité de commande 7 ouvre les vannes EV81 et EV831 et ferme les vannes EV82, EV832 et EV833. Lorsque le compresseur 8 est activé, l'air ambiant est aspiré au niveau de l'entrée 8e puis refoulé dans la ligne L8 par la sortie 8s. L'air circule dans les lignes L8 et L831 et sort de la machine M au niveau de la sortie 58s. L'air pénètre dans la première poche 481 au niveau de l'entrée 48e.

Dès que la pression dans la première poche 481 atteint la pression de consigne, le régulateur 80 informe l'unité de commande 7. Celle-ci ferme alors la vanne EV831 (de sorte que la première poche 481 reste gonflée) et ouvre la vanne EV832. L'air circule dans les lignes L8 et L832 de sorte que la deuxième poche 482 se gonfle.

Dès que la pression dans la deuxième poche 482 atteint la pression de consigne, le régulateur 80 informe l'unité de commande 7. Celle-ci ferme alors la vanne EV832 (de sorte que la deuxième poche 481 reste gonflée) et ouvre la vanne EV833. L'air circule dans les lignes L8 et L833 de sorte que la troisième poche 483 se gonfle.

Dès que la pression dans la troisième poche 483 atteint la pression de consigne, le régulateur 80 informe l'unité de commande 7 qui va piloter la vanne EV81 pour la mettre en position de purge et mettre à l'échappement ladite troisième poche. L'unité de commande 7 va ensuite ouvrir la vanne EV832 pour dégonfler la deuxième poche 482, puis ouvrir la vanne EV831 pour dégonfler la première poche 481.

Durant la séance de traitement, des nouveaux cycles de gonflage/dégonflage peuvent être relancés, ces cycles étant identiques ou différents.

Dans le mode de réalisation de la figure 12, le régulateur 80 peut être utilisé pour purger les poches 481, 482, 483 (auquel cas la vanne EV81 n'est pas nécessaire). Toutefois, le temps de purge peut être plus long. La vanne EV81 est donc préférentiellement utilisée pour avoir une purge franche et rapide.

Le dispositif illustré sur les figures 7 et 12 permet de traiter une même personne sur une ou plusieurs zones distinctes de son corps. L'enveloppe 4c peut par exemple être installée sur un bras pour un traitement par pressothérapie avec effet pompage et l'autre enveloppe 4d sur une cuisse pour un traitement par pressothérapie avec effet drainage. Bien évidement, il est possible de n'utiliser que l'enveloppe 4c ou que l'enveloppe 4d.

Il est également envisageable d'utiliser, pour une même personne, plusieurs enveloppes 4c pour un traitement par pressothérapie avec effet pompage. Dans ce cas, on duplique la ligne L82 (et la sortie 58s) d'autant d'enveloppes 4c que nécessaire.

Il est encore envisageable d'utiliser, pour une même personne, plusieurs enveloppes 4d pour un traitement par pressothérapie avec effet drainage. Dans ce cas, on duplique les séries de lignes L831, L832, L833, ... (et les séries de sorties 581s, 582s, 583s, ....) d'autant d'enveloppes 4d que nécessaire.

Le dispositif des figures 7 et 12 peut également être utilisé pour traiter plusieurs personnes sur une ou plusieurs zones de leur corps. Dans ce cas, on peut dupliquer l'ensemble du dispositif en utilisant une ou plusieurs autres pompes 8' connectées à un ou plusieurs autres circuits pneumatiques identiques à celui des figures 7 et 12 (c'est-à-dire avec régulateur de pression, et le même système de vannes).

On peut également conserver la pompe 8 qui est une pompe commune alimentant le ou les autres circuits pneumatiques. La pompe 8 est dans ce cas redimensionnée de manière à pouvoir supporter les contraintes de débit d'air et de pression d'air. Dans ce cas, on peut prévoir un ou plusieurs piquages au niveau de la sortie 8s de la pompe 8, servant à alimenter le ou les autres circuits pneumatiques. Un tel piquage est schématisé en pointillés sur les figures 7 et 12 et porte la référence L8'.

Selon des modes de réalisation non couverts par la présente invention, le dispositif de pressothérapie P est utilisé dans une machine de cryothérapie ayant une conception autre que celle revendiquée, ou dans une machine de pressothérapie qui n'est pas adaptée pour assurer un quelconque traitement par cryothérapie (c'est-à-dire dans une machine de pressothérapie seule).

La machine M selon l'invention a trois modes de fonctionnement possibles :
- un mode de fonctionnement par cryothérapie où seul le circuit hydraulique est utilisé,
- un mode de fonctionnement par pressothérapie où seul le circuit pneumatique est utilisé,
- un mode de fonctionnement hybride combinant la cryothérapie et la pressothérapie et où le circuit hydraulique et le circuit pneumatique sont simultanément utilisés.

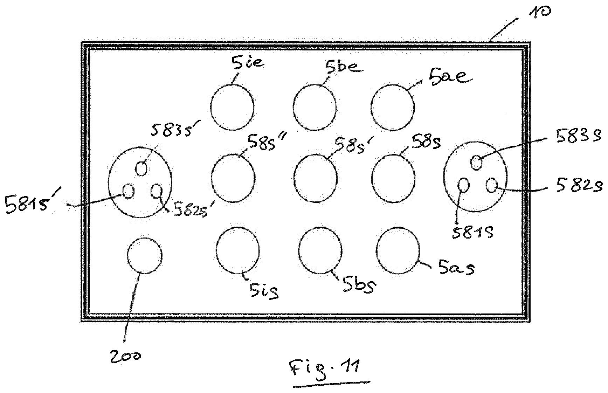

La figure 11 illustre une platine 10 pour la connexion des enveloppes sur la machine M. Cette platine 10 est située au niveau d'une face avant ou latérale de la machine M, dans une zone parfaitement accessible. La platine 10 regroupe l'ensemble des connecteurs permettant de connecter la ou les enveloppes au circuit hydraulique et au circuit pneumatique, cette configuration permettant de simplifier l'utilisation de la machine M pour le praticien.

Sur la figure 11, la platine 10 comporte :
- une série de connecteurs pour connecter les enveloppes au circuit hydraulique, et notamment :
   ∘ des connecteurs d'entrée 5ae, 5be, 5ie adaptés pour connecter les tuyaux reliés à la sortie des cheminements 40 des enveloppes pour cryothérapie,
   ∘ des connecteurs de sortie 5as, 5bs, 5is adaptés pour connecter les tuyaux reliés à l'entrée des cheminements 40 des enveloppes pour cryothérapie,

- une série de connecteurs pour connecter les enveloppes au circuit pneumatique, et notamment :
   ∘ des connecteurs 58s, 58s', 58s" adaptés pour connecter les tuyaux reliés aux poches des enveloppes pour pressothérapie avec effet pompage,
   ∘ un ou plusieurs connecteurs 581s, 582s, 583s, 581s', 582s', 583s' adaptés pour connecter les tuyaux reliés aux poches des enveloppes pour pressothérapie avec effet drainage.
- éventuellement un connecteur pour la mise sous pression du circuit hydraulique (lorsque celui-ci est un circuit fermé sous pression).

Les connecteurs 581s, 582s, 583s d'une part et 581s', 582s', 583s' d'autre part, se présente avantageusement sous la forme de multi-connecteurs. Ils permettent en un seul branchement, de connecter simultanément au circuit pneumatique toutes les poches d'une enveloppe pour pressothérapie avec effet drainage.

L'agencement des différents éléments et/ou moyens et/ou étapes de l'invention, dans les modes de réalisation décrits ci-dessus, ne doit pas être compris comme exigeant un tel agencement dans toutes les implémentations. Selon un mode de réalisation non couvert par les revendication, le circuit hydraulique n'est pas nécessairement un circuit fermé, mais pourrait être conçu comme un circuit ouvert.

## Revendications

1. Machine adaptée pour un traitement par cryothérapie d'une zone d'un corps humain ou animal, la machine (M) comportant :
- un réservoir (1) de liquide présentant une entrée (1e) et une sortie (1s),
- une pompe hydraulique (2) présentant une entrée (2e) d'aspiration du liquide et une sortie (2s) de refoulement du liquide, laquelle pompe est située en amont du réservoir (1)
- une unité de réfrigération (3) du liquide, laquelle unité comporte une entrée (3e) et une sortie (3s),
- un circuit hydraulique fermé sous pression comportant :
∘ une troisième ligne hydraulique (L3) reliant la sortie (1s) du réservoir (1) à l'entrée (2e) de la pompe (2),
∘ une quatrième ligne hydraulique (L4) reliant la sortie (1s) du réservoir (1) à la sortie (5s) dudit circuit,
∘ une cinquième ligne hydraulique (L5) reliant l'entrée (5e) dudit circuit à l'entrée (2e) de la pompe (2),
∘ une sortie (5s) par laquelle le fluide refroidi circule vers une enveloppe (4) adaptée pour être installée sur la zone à traiter,
∘ une entrée (5e) par laquelle le liquide retourne de l'enveloppe (4),
- une unité de commande (7) contrôlant le régime de la pompe (2) en fonction de signaux de pression émis par des capteurs de pression (62) installés dans le circuit hydraulique,
**se caractérisant par le fait que :**
- la pompe (2) est la seule pompe hydraulique connectée au circuit hydraulique,
- l'unité de réfrigération (3) est située en dehors du réservoir (1), laquelle unité de réfrigération comporte une entrée (3e) et une sortie (3s),
- le circuit hydraulique comporte :
∘ une première ligne hydraulique (L1) reliant la sortie (2s) de la pompe (2) à l'entrée (3e) de l'unité de réfrigération (3),
∘ une deuxième ligne hydraulique (L2) reliant la sortie (3s) de l'unité de réfrigération (3) à l'entrée (1e) du réservoir (1),
- au moins un vase (61, 610) d'expansion des variations de pression et/ou de volume du liquide dans le circuit hydraulique est intégré dans ledit circuit,
- l'unité de commande (7) contrôle le régime de la pompe (2) en fonction de signaux de pression émis par les capteurs de pression (62) installés dans le circuit hydraulique et/ou en fonction de signaux de débit émis par au moins un débitmètre installé dans ledit circuit hydraulique,

2. Machine selon la revendication 1, dans laquelle un système de vannes (EV3, EV4) est agencé dans le circuit hydraulique, de sorte que dans un état de fonctionnement desdites vannes : la quatrième ligne hydraulique (L4) est isolée et la troisième ligne hydraulique (L3) ouverte, le liquide circulant dans ladite machine (M), entre les première (L1), deuxième (L2), et troisième (L3) lignes hydrauliques.

3. Machine selon l'une des revendications précédentes, dans laquelle un système de vannes (EV3, EV4) est agencé dans le circuit hydraulique, de sorte que dans un état de fonctionnement desdites vannes : la troisième ligne hydraulique (L3) est isolée et la quatrième ligne hydraulique (L4) ouverte, le liquide circulant, dans ladite machine (M), dans les première (L1), deuxième (L2), quatrième (L4) et cinquième (L5) lignes hydrauliques.

4. Machine selon l'une des revendications précédentes, dans laquelle :
- le vase d'expansion (610) est à volume réglable et comporte un piston (613) dont la position est réglable pour moduler le volume et/ou la pression du liquide dans le circuit hydraulique,
- le piston (613) est actionné par un vérin (614), lequel vérin est piloté par une unité de commande (7) en fonction de mesures de pression réalisées au niveau de l'entrée (2e) et de la sortie (2s) de la pompe (2).

5. Machine selon la revendication 4, comportant l'une des caractéristiques suivantes :
- le vase (610) d'expansion est connecté dans une ligne hydraulique (L3, L5) reliée à l'entrée (2e) de la pompe (2),
- le vase (610) d'expansion est connecté dans la première ligne hydraulique (L1) reliée à la sortie (2s) de la pompe (2),
- un premier vase d'expansion (610) est connecté dans la cinquième ligne hydraulique (L5) et un seconde vase d'expansion (610) est connecté dans la première ligne hydraulique (L1).

6. Machine selon l'une des revendications précédentes, comportant en outre :
- un dispositif de pressothérapie (P) comportant au moins une pompe (8) alimentant un circuit pneumatique,
- le circuit pneumatique comporte au moins une ligne pneumatique (L8, L82, L831, L832, L833) pour connecter la sortie (8s) de la pompe pneumatique (8) à au moins une sortie (58s, 581s, 582s, 582s) adaptée pour mettre en communication pneumatique ledit circuit pneumatique avec au moins une poche gonflable (48, 481, 482, 483) d'une enveloppe (4c, 4d) adaptée pour être installée sur une zone à traiter.

7. Machine selon la revendication 6, dans laquelle le circuit pneumatique et le circuit hydraulique sont indépendants l'un de l'autre, ladite machine ayant trois modes de fonctionnement :
- un mode de fonctionnement par cryothérapie où seul le circuit hydraulique est utilisé,
- un mode de fonctionnement par pressothérapie où seul le circuit pneumatique est utilisé,
- un mode de fonctionnement combinant la cryothérapie et la pressothérapie et où le circuit hydraulique et le circuit pneumatique sont simultanément est utilisés.

8. Machine selon l'une des revendications 6 ou 7, dans laquelle le circuit pneumatique comporte au moins une ligne pneumatique (L8, L82) pour un traitement par pressothérapie avec effet pompage et/ou au moins une ligne pneumatique (L8, L831, L832, L833) pour un traitement par pressothérapie avec effet drainage.

9. Machine selon l'une des revendications précédentes, dans laquelle le circuit hydraulique comporte :
- plusieurs sorties (5as, 5bs) reliées à plusieurs branches (L4a, L4b) de la quatrième ligne hydraulique (L4) et par lesquelles le liquide refroidi circule depuis ladite quatrième ligne vers plusieurs enveloppes (4a, 4b),
- plusieurs entrées (5ae, 5be) reliées à la cinquième ligne hydraulique (L5) et par lesquelles le liquide refroidi circule depuis les enveloppes vers ladite cinquième ligne.

10. Machine selon la revendication 9, dans laquelle chaque branche (L4a, L4b) de la quatrième ligne hydraulique (L4) est pourvue d'une vanne (EV4a, EV4b), lesquelles vannes sont adaptées pour isoler ou ouvrir de manière indépendante les branches dans lesquelles elles sont respectivement installées.

11. Système de traitement par cryothérapie d'une zone d'un corps humain ou animal, le système comportant :
- une machine (M) adaptée pour un traitement par cryothérapie,
- au moins une enveloppe (4) adaptée pour être installée sur la zone à traiter,
**se caractérisant par le fait que :**
- la machine (M) est conforme à la revendication 1,
- l'enveloppe (4) est connectée à l'entrée (5e) et à la sortie (5s) du circuit hydraulique de la machine (M) de sorte que le liquide refroidi circule dans ladite enveloppe.

12. Procédé de fonctionnement d'une machine (M) conforme à la revendication 1, avant une séance de cryothérapie, le procédé comportant les étapes consistant à :
- déterminer une plage cible de températures du liquide, laquelle plage a une borne supérieure et une borne inférieure,
- mesurer la température du liquide dans le circuit hydraulique de la machine (M),
- comparer la température mesurée à la plage cible de température,
- faire fonctionner la machine (M) selon un mode de fonctionnement qui dépend du résultat de cette comparaison.

13. Procédé selon la revendication 12, dans lequel, si la température mesurée est égale ou supérieure à la borne supérieure, alors le procédé comporte les étapes consistant à :
- isoler la quatrième ligne hydraulique (L4), ouvrir la troisième ligne hydraulique (L3) et actionner la pompe (2) de sorte que le liquide circule en circuit fermé entre les première (L1), deuxième (L2) et troisième (L3) lignes hydrauliques,
- actionner l'unité de réfrigération (3) de manière refroidir le liquide.

14. Procédé selon l'une des revendications 12 ou 13, dans lequel, si la température mesurée est comprise entre la borne inférieure et la borne supérieure, alors le procédé comporte les étapes consistant à désactiver l'unité de réfrigération (3) et désactiver la pompe (2) pour empêcher le refroidissement et la circulation du liquide dans le circuit hydraulique.

15. Procédé de fonctionnement d'un système conforme à la revendication 11, pour un usage non-thérapeutique, comportant les étapes consistant à :
- isoler la troisième ligne hydraulique (L3), ouvrir la quatrième ligne (L4) et actionner la pompe (2) de sorte que le liquide circule en circuit fermé entre la première ligne hydraulique (L1), la deuxième ligne hydraulique (L2), la quatrième ligne hydraulique (L4), l'enveloppe (4) et la cinquième ligne hydraulique (L5),
- déterminer une plage de températures de traitement du liquide, laquelle plage a une borne supérieure et une borne inférieure,
- mesurer la température du liquide dans le circuit hydraulique de la machine (M),
- comparer la température mesurée à la plage de températures de traitement,
∘ si la température mesurée est égale ou supérieure à la borne supérieure alors : actionner l'unité de réfrigération (3) de manière à refroidir le liquide,
∘ si la température mesurée est comprise entre la borne inférieure et la borne supérieure alors : désactiver l'unité de réfrigération (3) pour ne pas refroidir le liquide.

## Patentansprüche

1. Maschine, die zur Behandlung mit Kryotherapie eines Bereichs eines menschlichen oder tierischen Körpers geeignet ist, wobei die Maschine (M) umfasst:
- einen Behälter (1) für Flüssigkeit, der einen Einlass (1e) und einen Auslass (1s) aufweist,
- eine Hydraulikpumpe (2), die einen Einlass (2e) zum Ansaugen der Flüssigkeit und einen Auslass (2s) zum Ausstoßen der Flüssigkeit aufweist, wobei die Pumpe stromauf des Behälters (1) gelegen ist,
- eine Kühleinheit (3) zum Kühlen der Flüssigkeit, wobei die Einheit einen Einlass (3e) und einen Auslass (3s) umfasst,
- einen unter Druck stehenden geschlossenen Hydraulikkreislauf, umfassend:
∘ eine dritte Hydraulikleitung (L3), die den Auslass (1s) des Behälters (1) mit dem Einlass (2e) der Pumpe (2) verbindet,
∘ eine vierte Hydraulikleitung (L4), die den Auslass (1s) des Behälters (1) mit dem Auslass (5s) des Kreislaufs verbindet,
∘ eine fünfte Hydraulikleitung (L5), die den Einlass (5e) des Kreislaufs mit dem Einlass (2e) der Pumpe (2) verbindet,
∘ einen Auslass (5s), durch den die gekühlte Flüssigkeit zu einer Ummantelung (4) zirkuliert, die geeignet ist, auf dem zu behandelnden Bereich angebracht zu werden,
∘ einen Einlass (5e), durch den die Flüssigkeit von der Ummantelung (4) zurückfließt,
- eine Steuereinheit (7), die die Leistung der Pumpe (2) in Abhängigkeit von Drucksignalen steuert, die von im Hydraulikkreislauf angebrachten Drucksensoren (62) ausgegeben werden,
**dadurch gekennzeichnet, dass**:
- die Pumpe (2) die einzige Hydraulikpumpe ist, die an den Hydraulikkreislauf angeschlossen ist,
- die Kühleinheit (3) außerhalb des Behälters (1) gelegen ist, wobei die Einheit einen Einlass (3e) und einen Auslass (3s) umfasst,
- der Hydraulikkreislauf umfasst:
∘ eine erste Hydraulikleitung (L1), die den Auslass (2s) der Pumpe (2) mit dem Einlass (3e) der Kühleinheit (3) verbindet,
∘ eine zweite Hydraulikleitung (L2), die den Auslass (3s) der Kühleinheit (3) mit dem Einlass (1e) des Behälters (1) verbindet,
- mindestens ein Ausdehnungsgefäß (61, 610) für Druck- und/oder Volumenänderungen der Flüssigkeit im Hydraulikkreislauf in den Kreislauf integriert ist,
- die Steuereinheit (7) die Leistung der Pumpe (2) in Abhängigkeit von Drucksignalen steuert, die von im Hydraulikkreislauf installierten Drucksensoren (62) ausgegeben werden, oder in Abhängigkeit von Durchflusssignalen, die von mindestens einem im Hydraulikkreislauf installierten Durchflussmesser ausgegeben werden.

2. Maschine nach Anspruch 1, bei der ein Ventilsystem (EV3, EV4) im Hydraulikkreislauf angeordnet ist, so dass in einem Betriebszustand der Ventile die vierte Hydraulikleitung (L4) abgesperrt ist und die dritte Hydraulikleitung (L3) offen ist, wobei die Flüssigkeit in der Maschine (M) zwischen den ersten (L1), zweiten (L2) und dritten (L3) Hydraulikleitungen zirkuliert.

3. Maschine nach einem der vorhergehenden Ansprüche, bei der ein Ventilsystem (EV3, EV4) im Hydraulikkreislauf angeordnet ist, so dass in einem Betriebszustand der Ventile die dritte Hydraulikleitung (L3) abgesperrt ist und die vierte Hydraulikleitung (L4) offen ist, wobei die Flüssigkeit in der Maschine (M) in den ersten (L1), zweiten (L2), vierten (L4) und fünften (L5) Hydraulikleitungen zirkuliert.

4. Maschine nach einem der vorhergehenden Ansprüche, bei der:
- das Ausdehnungsgefäß (610) ein regelbares Volumen hat und einen Kolben (613) umfasst, dessen Position regelbar ist, um das Volumen und/oder den Druck der Flüssigkeit im Hydraulikkreislauf anzupassen,
- der Kolben (613) von einem Zylinder (614) betätigt wird, wobei der Zylinder von einer Steuereinheit (7) in Abhängigkeit von Druckmessungen angesteuert wird, die am Einlass (2e) und Auslass (2s) der Pumpe (2) ausgeführt werden.

5. Maschine nach Anspruch 4, die eines der folgenden Merkmale umfasst:
- das Ausdehnungsgefäß (610) ist in eine Hydraulikleitung (L3, L5) eingebaut, die mit dem Einlass (2e) der Pumpe (2) verbunden ist,
- das Ausdehnungsgefäß (610) ist in die erste Hydraulikleitung (L1) eingebaut, die mit dem Auslass (2s) der Pumpe (2) verbunden ist,
- ein erstes Ausdehnungsgefäß (610) ist in die fünfte Hydraulikleitung (L5) eingebaut und ein zweites Ausdehnungsgefäß (610) ist in die erste Hydraulikleitung (L1) eingebaut.

6. Maschine nach einem der vorhergehenden Ansprüche, die ferner umfasst:
- eine Pressotherapievorrichtung (P), die mindestens eine Pumpe (8) umfasst, die einen Pneumatikkreislauf versorgt,
- der Pneumatikkreislauf umfasst mindestens eine Pneumatikleitung (L8, L82, L831, L832, L833), um den Auslass (8s) der Pneumatikpumpe (8) an mindestens einen Auslass (58s, 581s, 582s, 582s) anzuschließen, der geeignet ist, eine pneumatische Verbindung des Pneumatikkreislaufs mit mindestens einem aufblasbaren Beutel (48, 481, 482, 483) einer Ummantelung (4c, 4d) herzustellen, die geeignet ist, auf einem zu behandelnden Bereich angebracht zu werden.

7. Maschine nach Anspruch 6, bei der der Pneumatikkreislauf und der Hydraulikkreislauf voneinander unabhängig sind, wobei die Maschine drei Betriebsarten hat:
- eine Betriebsart mit Kryotherapie, bei der nur der Hydraulikkreislauf verwendet wird,
- eine Betriebsart mit Pressotherapie, bei der nur der Pneumatikkreislauf verwendet wird,
- eine Betriebsart, bei der Kryotherapie und Pressotherapie kombiniert sind und der Hydraulikkreislauf und der Pneumatikkreislauf gleichzeitig verwendet werden.

8. Maschine nach einem der Ansprüche 6 oder 7, bei der der Pneumatikkreislauf mindestens eine Pneumatikleitung (L8, L82) für eine Behandlung mit Pressotherapie mit Pumpwirkung und/oder mindestens eine Pneumatikleitung (L8, L831, L832, L833) für eine Behandlung mit Pressotherapie mit Drainagewirkung umfasst.

9. Maschine nach einem der vorhergehenden Ansprüche, bei der der Hydraulikkreislauf umfasst:
- mehrere Auslässe (5as, 5bs), die mit mehreren Zweigen (L4a, L4b) der vierten Hydraulikleitung (L4) verbunden sind und durch die die gekühlte Flüssigkeit von der vierten Leitung zu mehreren Ummantelungen (4a, 4b) zirkuliert,
- mehrere Einlässe (5ae, 5be), die mit der fünften Hydraulikleitung (L5) verbunden sind und durch die die gekühlte Flüssigkeit von den Ummantelungen zur fünften Leitung zirkuliert.

10. Maschine nach Anspruch 9, bei der jeder Zweig (L4a, L4b) der vierten Hydraulikleitung (L4) mit einem Ventil (EV4a, EV4b) versehen ist, wobei die Ventile geeignet sind, die Zweige, in denen sie jeweils installiert sind, unabhängig abzusperren und zu öffnen.

11. System zur Behandlung mit Kryotherapie eines Bereichs eines menschlichen oder tierischen Körpers, wobei das System umfasst:
- eine Maschine (M), die zur Behandlung mit Kryotherapie geeignet ist,
- mindestens eine Ummantelung (4), die geeignet, auf dem zu behandelnden Bereich angebracht zu werden,
**dadurch gekennzeichnet, dass**:
- die Maschine (M) Anspruch 1 entspricht,
- die Ummantelung (4) an den Einlass (5e) und an den Auslass (5s) des Hydraulikkreislaufs der Maschine (M) angeschlossen ist, so dass die gekühlte Flüssigkeit in der Ummantelung zirkuliert.

12. Verfahren zum Betreiben einer Maschine (M) nach Anspruch 1 vor einer Kryotherapiesitzung, wobei das Verfahren die Schritte umfasst, die darin bestehen:
- einen Temperatur-Zielbereich für die Flüssigkeit zu bestimmen, wobei der Bereich einen oberen Grenzwert und einen unteren Grenzwert hat,
- die Temperatur der Flüssigkeit im Hydraulikkreislauf der Maschine (M) zu messen,
- die gemessene Temperatur mit dem Temperatur-Zielbereich zu vergleichen,
- die Maschine (M) gemäß einer Betriebsart zu betreiben, die vom Ergebnis dieses Vergleichs abhängt.

13. Verfahren nach Anspruch 12, bei dem, wenn die gemessene Temperatur größer oder gleich dem oberen Grenzwert ist, das Verfahren die Schritte umfasst, die darin bestehen:
- die vierte Hydraulikleitung (L4) abzusperren, die dritte Hydraulikleitung (L3) zu öffnen und die Pumpe (2) zu betätigen, so dass die Flüssigkeit im geschlossenen Kreislauf zwischen den ersten (L1), zweiten (L2) und dritten (L3) Hydraulikleitungen zirkuliert,
- die Kühleinheit (3) zu betätigen, so dass die Flüssigkeit gekühlt wird.

14. Verfahren nach einem der Ansprüche 12 oder 13, bei dem, wenn die gemessene Temperatur zwischen dem unteren Grenzwert und dem oberen Grenzwert liegt, das Verfahren die Schritte umfasst, die darin bestehen, die Kühleinheit (3) zu deaktivieren und die Pumpe (2) zu deaktivieren, um das Kühlen und das Zirkulieren der Flüssigkeit im Hydraulikkreislauf zu verhindern.

15. Verfahren zum Betreiben eines Systems nach Anspruch 11 zu einer nicht-therapeutischen Verwendung, umfassend die Schritte, die darin bestehen:
- die dritte Hydraulikleitung (L3) abzusperren, die vierte Leitung (L4) zu öffnen und die Pumpe (2) zu betätigen, so dass die Flüssigkeit im geschlossenen Kreislauf zwischen der ersten Hydraulikleitung (L1), der zweiten Hydraulikleitung (L2), der vierten Hydraulikleitung (L4), der Ummantelung (4) und der fünften Hydraulikleitung (L5) zirkuliert,
- einen Behandlungstemperaturbereich der Flüssigkeit zu bestimmen, wobei der Bereich einen oberen Grenzwert und einen unteren Grenzwert hat,
- die Temperatur der Flüssigkeit im Hydraulikkreislauf der Maschine (M) zu messen,
- die gemessene Temperatur mit dem Behandlungstemperaturbereich zu vergleichen,
∘ wenn die gemessene Temperatur größer oder gleich dem oberen Grenzwert ist, die Kühleinheit (3) zu betätigen, so dass die Flüssigkeit gekühlt wird,
∘ wenn die gemessene Temperatur zwischen dem unteren Grenzwert und dem oberen Grenzwert liegt, die Kühleinheit (3) zu deaktivieren, um die Flüssigkeit nicht zu kühlen.

## Claims

1. Machine suitable for cryotherapy treatment of a zone of a human or animal body, the machine (M) comprising:
- a tank (1) of liquid having an inlet (1e) and an outlet (1s),
- a hydraulic pump (2) having a liquid suction inlet (2e) and a liquid delivery outlet (2s), which pump is situated upstream of the tank (1),
- a liquid refrigeration unit (3), which unit comprises an inlet (3e) and an outlet (3s),
- a pressurized closed hydraulic circuit comprising:
∘ a third hydraulic line (L3) connecting the outlet (1s) of the tank (1) to the inlet (2e) of the pump (2),
∘ a fourth hydraulic line (L4) connecting the outlet (1s) of the tank (1) to the outlet (5s) of said circuit,
∘ a fifth hydraulic line (L5) connecting the inlet (5e) of said circuit to the inlet (2e) of the pump (2),
∘ an outlet (5s) via which the cooled liquid circulates towards a wrap (4) suitable for being fitted onto the zone that is to be treated,
∘ an inlet (5e) via which the liquid returns from the wrap (4),
- a control unit (7) controlling the speed of the pump (2) on the basis of pressure signals emitted by pressure sensors (62) installed in the hydraulic circuit,
**characterized in that**:
- the pump (2) is the only hydraulic pump connected to the hydraulic circuit,
- the refrigeration unit (3) is situated outside of the tank (1), which refrigeration unit comprises an inlet (3e) and an outlet (3s),
- the hydraulic circuit comprises:
∘ a first hydraulic line (L1) connecting the outlet (2s) of the pump (2) to the inlet (3e) of the refrigeration unit (3),
∘ a second hydraulic line (L2) connecting the outlet (3s) of the refrigeration unit (3) to the inlet (1e) of the tank (1),
- at least one expansion vessel (61, 610) for absorbing the variations in pressure and/or in volume of the liquid in the hydraulic circuit is incorporated into said circuit,
- the control unit (7) controls the speed of the pump (2) on the basis of pressure signals emitted by the pressure sensors (62) installed in the hydraulic circuit and/or on the basis of flow rate signals emitted by at least one flow meter installed in said hydraulic circuit.

2. Machine according to Claim 1, wherein a system of valves (EV3, EV4) is arranged in the hydraulic circuit so that in one state of operation of said valves: the fourth hydraulic line (L4) is isolated and the third hydraulic line (L3) is open, the liquid circulating in said machine (M) between the first (L1), second (L2) and third (L3) hydraulic lines.

3. Machine according to one of the preceding claims, wherein a system of valves (EV3, EV4) is arranged in the hydraulic circuit so that in one state of operation of said valves: the third hydraulic line (L3) is isolated and the fourth hydraulic line (L4) is open, the liquid circulating, in said machine (M), in the first (L1), second (L2), fourth (L4) and fifth (L5) hydraulic lines.

4. Machine according to one of the preceding claims, wherein:
- the expansion vessel (610) is a variable-volume vessel and comprises a piston (613) of which the position is adjustable so as to modify the volume and/or the pressure of the liquid in the hydraulic circuit,
- the piston (613) is actuated by a ram (614), which ram is controlled by a control unit (7) on the basis of pressure measurements taken at the inlet (2e) and at the outlet (2s) of the pump (2) .

5. Machine according to Claim 4, comprising one of the following features:
- the expansion vessel (610) is connected in a hydraulic line (L3, L5) which is connected to the inlet (2e) of the pump (2),
- the expansion vessel (610) is connected in the first hydraulic line (L1) which is connected to the outlet (2s) of the pump (2),
- a first expansion vessel (610) is connected in the fifth hydraulic line (L5) and a second expansion vessel (610) is connected in the first hydraulic line (L1).

6. Machine according to one of the preceding claims, further comprising:
- a pressure-therapy device (P) comprising at least one pump (8) supplying a pneumatic circuit,
- the pneumatic circuit comprises at least one pneumatic line (L8, L82, L831, L832, L833) for connecting the outlet (8s) of the pneumatic pump (8) to at least one outlet (58s, 581s, 582s, 582s) suitable for placing said pneumatic circuit in pneumatic communication with at least one inflatable pocket (48, 481, 482, 483) of a wrap (4c, 4d) suitable for being fitted on a zone that is to be treated.

7. Machine according to Claim 6, wherein the pneumatic circuit and the hydraulic circuit are independent of one another, said machine having three modes of operation:
- a cryotherapy mode of operation in which only the hydraulic circuit is used,
- a pressure-therapy mode of operation in which only the pneumatic circuit is used,
- a mode of operation that combines cryotherapy with pressure therapy and in which the hydraulic circuit and the pneumatic circuit are simultaneously used.

8. Machine according to one of Claims 6 and 7, wherein the pneumatic circuit comprises at least one pneumatic line (L8, L82) for a pressure-therapy treatment with a pumping effect and/or at least one pneumatic line (L8, L831, L832, L833) for a pressure-therapy treatment with a draining effect.

9. Machine according to one of the preceding claims, wherein the hydraulic circuit comprises:
- a plurality of outlets (5as, 5bs) connected to a plurality of branches (L4a, L4b) of the fourth hydraulic line (L4) and via which the cooled liquid circulates from said fourth line to a plurality of wraps (4a, 4b),
- a plurality of inlets (5ae, 5be) connected to the fifth hydraulic line (L5) and via which the cooled liquid circulates from the wraps to said fifth line.

10. Machine according to Claim 9, wherein each branch (L4a, L4b) of the fourth hydraulic line (L4) is provided with a valve (EV4a, EV4b), which valves are suitable for isolating or opening, independently, the branches in which they are respectively installed.

11. System for the cryotherapy treatment of a zone of a human or animal body, the system comprising:
- a machine (M) suitable for cryotherapy treatment,
- at least one wrap (4) suitable for being fitted on the zone that is to be treated,
**characterized in that**:
- the machine (M) is in accordance with Claim 1,
- the wrap (4) is connected to the inlet (5e) and to the outlet (5s) of the hydraulic circuit of the machine (M) so that the cooled liquid circulates in said wrap.

12. Method for operating a machine (M) according to Claim 1, prior to a cryotherapy session, the method comprising the steps consisting in:
- determining a target range for the temperature of the liquid, which range has an upper boundary and a lower boundary,
- measuring the temperature of the liquid in the hydraulic circuit of the machine (M),
- comparing the measured temperature with the target temperature range,
- operating the machine (M) in a mode of operation that is dependent on the result of this comparison.

13. Method according to Claim 12, wherein, if the measured temperature is equal to or higher than the upper boundary, then the method comprises the steps consisting in:
- isolating the fourth hydraulic line (L4), opening the third hydraulic line (L3) and actuating the pump (2) so that the liquid circulates in a closed circuit between the first (L1), second (L2) and third (L3) hydraulic lines,
- actuating the refrigeration unit (3) in such a way as to cool the liquid.

14. Method according to one of Claims 12 and 13, wherein, if the measured temperature is comprised between the lower boundary and the upper boundary, then the method comprises the steps consisting in deactivating the refrigeration unit (3) and deactivating the pump (2) so as to prevent the cooling and circulation of the liquid in the hydraulic circuit.

15. Method of operating a system according to Claim 11, for a non-therapeutic use, comprising the steps consisting in:
- isolating the third hydraulic line (L3), opening the fourth line (L4) and actuating the pump (2) so that the liquid circulates in closed-circuit between the first hydraulic line (L1), the second hydraulic line (L2), the fourth hydraulic line (L4), the wrap (4) and the fifth hydraulic line (L5),
- determining a range of treatment temperatures for the liquid, which range has an upper boundary and a lower boundary,
- measuring the temperature of the liquid in the hydraulic circuit of the machine (M),
- comparing the measured temperature with the range of treatment temperatures,
∘ if the temperature is equal to or higher than the upper boundary, then: actuating the refrigeration unit (3) in such a way as to cool the liquid,
∘ if the measured temperature is comprised between the lower boundary and the upper boundary, then: deactivating the refrigeration unit (3) so as not to cool the liquid.
